# EUROPEAN PATENT APPLICATION

(11) **EP 2 532 754 A1**
(43) Date of publication of application: **12.12.2012**
(21) Application number: 11168864.4
(22) Date of filing: 07.06.2011
(51) Int. Cl.: C12Q 1/68, B01J 19/00

(54) **Devices and methods for efficient capture of nucleic acids**

(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

The present invention relates a device for the efficient binding of nucleic acids on a microarray, comprising a reaction zone comprising a microarray, and a capture zone comprising a porous membrane substrate, wherein the capture zone is capable of specifically capturing sense strands of target molecules, whereas complementary antisense strands are captured in the reaction zone, or vice versa. The invention further envisages temperature regulating units in the device allowing to bring or keep the capture at a temperature not allowing hybridizing or binding of nucleic acid(s) to the capture molecules or to a temperature suitable for nucleic acid hybridization. The invention also relates to a method of efficiently binding nucleic acids on a microarray, comprising introducing a medium containing one or more target molecules in denatured form into a capture zone of a device of the present invention, performing an interaction reaction between the target molecules and immobilized capture molecules in said capture zone, and transporting not bound target molecule strands to a reaction zone comprising a microarray, thereby allowing an interaction between the target molecule strands and immobilized capture molecules on the microarray. In a further aspect the invention relates to the use of such a device for enriching target molecules, specifically selecting a target molecules, expression analysis, comparative genomic hybridization, the detection of SNPs, or for microarray-based genomic selection-based sequencing.

## Description

### FIELD OF THE INVENTION

The present invention relates a device for the efficient binding of nucleic acids on a microarray, comprising a reaction zone comprising a microarray, and a capture zone comprising a porous membrane substrate, wherein the capture zone is capable of specifically capturing sense strands of target molecules, whereas complementary antisense strands are captured in the reaction zone, or vice versa. The invention further envisages temperature regulating units in the device allowing to bring or keep the capture at a temperature not allowing hybridizing or binding of nucleic acid(s) to the capture molecules or to a temperature suitable for nucleic acid hybridization. The invention also relates to a method of efficiently binding nucleic acids on a microarray, comprising introducing a medium containing one or more target molecules in denatured form into a capture zone of a device of the present invention, performing an interaction reaction between the target molecules and immobilized capture molecules in said capture zone, and transporting not bound target molecule strands to a reaction zone comprising a microarray, thereby allowing an interaction between the target molecule strands and immobilized capture molecules on the microarray. In a further aspect the invention relates to the use of such a device for enriching target molecules, specifically selecting a target molecules, expression analysis, comparative genomic hybridization, the detection of SNPs, or for microarray-based genomic selection-based sequencing.

### BACKGROUND OF THE INVENTION

Since the NIH project to initiate the sequencing of the whole human genome at the end of the 1990's, sequencing technology has evolved rapidly. Especially since the introduction of 2^{nd} generation of sequencing machines in 2005 the costs of sequencing have been reduced by a factor 10 to around 1 million US $ per human genome at the beginning of 2008. The sequencing industry is now aiming at reducing the costs of DNA sequencing even further with the aim of reaching costs of around 1000 US $ per human genome in the near future. Based on these prospects and expectations, DNA sequencing, in particular the sequencing of genomic DNA, as well as multiplexed detection or quantification of DNA sequences are seen as crucial diagnostic and research tools, which may be employed for the analysis of genetic variations, the detection of diseases or the elucidation of a predisposition for diseases. A prerequisite for all these approaches is the efficient isolation or capture of target DNA.

Binding of target DNA may be carried out by hybridization to a capture probe having a sequence complementary to the target sequence. Typically, capture probes are attached to or immobilized on suitable substrate surfaces. The efficiency of capture by hybridization depends on several parameters, the most important being the concentration of targets. In many cases the amount and/or concentration of target material is rather limited or amplification is difficult or undesired. In such situations, the efficiency of binding is of elevated importance and determines the limit of detection, the dynamic range and the accuracy of the analysis.

Since complex eukaryotic genomes like the human genome, are too large to be explored directly, complexity reduction methods are required. These are, for instance, based on the direct amplification of specific sequences by PCR methods, fosmid library construction, TAR cloning, or by microarray-based genomic selection (MGS), which has been developed to isolate user-defined unique genomic sequences from complex eukaryotic genomes (WO 2008/097887). This method encompasses physical shearing of genomic DNA to create random fragments of an average size of around 300 bp, an end-repairing of the fragments, a ligation to unique adaptors with complementary T nucleotide overhangs and the hybridization of the fragments to a high-density oligonucleotid microarray of complementary sequences indentified from a reference genome sequence, the subsequent elution of the fragments and their amplification via PCR using the adaptor sequences (WO 2008/097887). However, with current MGS protocols only about 80-90% of the target regions can be recovered. Thus, 10% to 20% of the target sequences are missing and several other regions may be covered only at a low level, which may impede a reliable discovery of mutations, e.g. in re-sequencing approaches. This finding may be explained by the fact that in typical hybridization mixtures both complementary strands are present with high copy numbers and thus favor a back-hybridization to the complementary strand instead of a binding to the capture probes.

There is, hence a need for improved means and methods for an efficient capturing of target nucleic acid molecules, in particular of target DNA molecules such as genomic nucleic acids, allowing for a reliable and quantitative recovery of the target molecules.

### SUMMARY OF THE INVENTION

The present invention addresses this need and provides means and methods, which allow an efficient capture of target nucleic acid molecules on microarrays. The objective is, in particular, accomplished by a device for the efficient binding of nucleic acids on a microarray, comprising:
A device for the efficient binding of nucleic acids on a microarray, comprising:
   (a) at least one reaction zone comprising a microarray, wherein the microarray comprises a substrate, on which one or more species of capture molecules are immobilized, and
   (b) at least one capture zone comprising a porous membrane substrate, on which one or more species of capture molecules are immobilized, which is in fluid connection with the reaction zone;
wherein said capture zone is capable of specifically capturing (a) sense strand(s) or part of (a) sense strand (s) of (a) target molecule(s), whereas (a) complementary antisense strand(s) or part of (an) antisense strand (s) of said target molecule(s) can be captured in said reaction zone; or wherein said capture zone is capable of specifically capturing (an) antisense strand(s) or part of (an) antisense strand (s) of said target molecule(s), whereas (a) complementary sense strand(s) or part of (a) sense strand (s) of said target molecule(s) can be captured in said reaction zone.

This device format and its use provide the advantage that the capture efficiency of nucleic acids can be drastically enhanced by nucleic acid strand separation. Thus, if one strand of a target molecule is specifically captured in a capture zone of the device, the counter-strand is free to bind to specific capture molecules on a microarray in adjacent reaction zones. Thereby re-hybridization of strands and counter-strands of nucleic acid molecules in the hybridization solution at the reaction zone can be prevented. Thus, the effective concentration of single stranded target molecules is drastically increased, which in turn increases the hybridization and the total capture rate. Hybridization steps can accordingly become orders of magnitude faster. In particular, by having in the capture zone(s) a large surface area in close contact with the sample and an excess of capture probes the binding equilibrium can be shifted to short times. Thereby the hybridization solution can be exhausted before a back-hybridization in solution occurs. Since the nucleic acid strand, which is captured on the porous membrane substrate, is depleted form solution very quickly, the hybridization of the counter-strand to microarrays in reaction zones will not be hindered by competing hybridization in solution. This type of reactions is not possible on the microarray since the concentration of capture probes is low and the accessibility is limited to a small region. Thus, by combining efficient capture on porous membrane substrate with high resolution capture on microarrays a new and very efficient capturing and separation approach is created. Captured molecules can subsequently be used for different purposes, inter alia for amplification reactions, labeling reactions, ligations, the performance of base extensions or for sequencing activities.

In a further preferred embodiment of the present invention the above described device comprises at least two capture zones, wherein one capture zone is capable of specifically capturing (a) sense strand(s) or part of (a) sense strand (s) of (a) target molecule(s), and the other capture zone is capable of specifically capturing (a) complementary antisense strand(s) or part of (an) antisense strand (s) of (a) target molecule(s).

In yet another preferred embodiment of the present invention said device comprises at least one temperature control and/or regulating unit for controlling and/or regulating the temperature within the device. In a particularly preferred embodiment said temperature control and/or regulating unit for controlling and/or regulating the temperature is located in a capture zone for controlling and/or regulating the temperature within said capture zone. In a further preferred embodiment, said temperature control and/or regulating unit for controlling and/or regulating the temperature is located in a capture zone and in a reaction zone for controlling and/or regulating the temperature within said capture zone or reaction zone, respectively.

In a further preferred embodiment of the present invention said device comprises at least two capture zones, wherein one of the at least two capture zones is kept at or brought to a melting temperature or a temperature not allowing hybridizing or binding of the nucleic acid(s) to the capture molecules, whereas another capture zone is kept at or brought to a temperature allowing hybridizing or binding of the nucleic acid(s) to the capture molecules. In a particularly preferred embodiment said melting temperature or temperature not allowing hybridizing or binding of the nucleic acid(s) to the capture molecules is a temperature of about 85°C to 98°C. In another particularly preferred embodiment, said temperature allowing hybridizing or binding of the nucleic acid(s) to the capture molecules is a temperature of about 45°C to 65°C.

In another preferred embodiment of the present invention said reaction zone comprises (a) capture molecule(s) complementary to the sense and anti-sense strand(s) or parts thereof of the target molecule(s). Alternatively, said reaction zone comprises (a) capture molecule(s) complementary to only the sense strand(s) or part of (a) sense strand (s) of the target molecule(s). In a further, alternative embodiment, said reaction zone may comprise (a) capture molecule(s) complementary to only the antisense strand(s) or part of (an) antisense strand (s) of the target molecule(s).

In another preferred embodiment of the present invention said capture zone comprises at least (a) capture molecule(s) complementary to the capture molecule(s) or a part thereof in said reaction zone. Alternatively, said capture zone may comprise a subset of capture molecules complementary to the capture molecule(s) or a part thereof in said reaction zone. In yet another preferred embodiment said capture zone may comprise or may additionally comprise (a) capture molecule(s) capable of binding to (an) interfering sequence(s). In yet another preferred embodiment the device according to the present invention, in particular as described herein above, may comprises at least two reaction zones wherein one reaction zone is capable of specifically binding a sense strand(s) or part of (a) sense strand (s) of (a) target molecule(s), and the other reaction zone is capable of specifically binding (a) complementary antisense strand(s) or part of (an) antisense strand (s) of (a) target molecule(s).

In a further preferred embodiment of the present invention said reaction zone(s) and said capture zone(s) as described herein above are arranged in a closed loop. In a particularly preferred embodiment said reaction zone(s) and said capture zone(s) as described herein above are arranged in an integrated device with said reaction zone(s) and said capture zone(s) in a connected microfluidic system. In an alternative embodiment of the present invention, said device may comprises two reaction zones and two capture zones, wherein a combination of one reaction zone and one capture zone is not in fluid connection with a combination of a second reaction zone and a second capture zone and wherein each reaction zone and each capture zone comprises (a) capture molecule(s) complementary to the capture molecule(s) of the other reaction zone or capture zone.

In yet another preferred embodiment of the present invention said capture molecule as described herein above has a length of about 20 to 80 nucleotides. In a particularly preferred embodiment said capture molecule has a length of about 40 to 65 nucleotides. In yet another particularly preferred embodiment said capture molecule has a length of about 60 nucleotides.

In a further preferred embodiment of the present invention said porous membrane substrate as described herein above is a fabric or felt. In a further embodiment, it may comprise or may additionally comprise a random porous structure. A preferred random porous structure would be nylon. In a further alternative embodiment, said porous membrane substrate may comprise a regular porous structure. In a further preferred embodiment said porous membrane substrate may comprise 3-dimensional and/or 2-dimensional pore structures. Particularly preferred are anisotropically etched Al-oxide, or electro-spun fibers. In another preferred embodiment, said porous membrane substrate may be created in situ. Particularly preferred is an in situ generation process by polymerization induced phase separation.

In a further aspect the present invention relates to a method of efficiently binding nucleic acids on a microarray, comprising the steps of:
(a) introducing a medium containing one or more target molecules in denatured form into a capture zone of a device as defined herein above;
(b) performing an interaction reaction between said target molecule(s) or a part thereof and immobilized capture molecule(s) in said capture zone, wherein (a) sense strand(s) or part of (a) sense strand (s) of (a) target molecule(s) is bound to said capture molecule(s), whereas (a) complementary antisense strand(s) or part of (an) antisense strand (s) of said target molecule(s) is not captured in said capture zone; or wherein (an) antisense strand(s) or part of (an) antisense strand (s) of said target molecule(s) is bound to said capture molecule(s), whereas (a) complementary sense strand(s) or part of (a) sense strand (s) of said target molecule(s) is not captured in said capture zone; and
(c) transporting not bound target molecule strand(s) to a reaction zone comprising a microarray, thereby allowing an interaction between said target molecule strand(s) or a part thereof and (an) immobilized capture molecule(s) on the microarray.

In a preferred embodiment of the present invention said method additionally comprising as step (d) detecting the interaction between said target molecule strand(s) or a part thereof and (an) immobilized capture molecule(s) on the microarray. In a particularly preferred embodiment said detection of the interaction is carried out via or based on the use of an optical read out system.

In a further preferred embodiment of the present invention, said interaction reaction between said target molecule(s) and immobilized capture molecule(s) as described herein above is performed in at least two capture zones wherein one capture zone is capable of specifically capturing a sense strand(s) or part of (a) sense strand (s) of (a) target molecule(s), and the other capture zone is capable of specifically capturing (a) complementary antisense strand(s) or part of (an) antisense strand (s) of (a) target molecule(s) and wherein one capture zones is kept at or brought to a melting temperature or a temperature not allowing hybridizing or binding of the nucleic acid(s) to the capture molecule(s), whereas another capture zone is kept at or brought to a temperature allowing hybridizing or binding of the nucleic acid(s) to the capture molecule(s). In a particularly preferred embodiment of the present invention said melting temperature or temperature not allowing hybridizing or binding of the nucleic acid(s) to the capture molecules is a temperature of about 85°C to 98°C. In another particularly preferred embodiment said temperature allowing hybridizing or binding of the nucleic acid(s) to the capture molecules is a temperature of about 45°C to 65°C.

In another preferred embodiment of the present invention, the herein above described method additionally comprises the step of a temperature switching between said at least two capture zones, wherein the temperature of the capture zone with a temperature not allowing hybridizing or binding of the nucleic acids to the capture molecules is lowered to a temperature allowing hybridizing or binding of the nucleic acids to the capture molecules, preferably a temperature of about 45°C to 65°C, and wherein the temperature of the capture zone with a temperature allowing hybridizing or binding of the nucleic acid(s) to the capture molecules is raised to a temperature not allowing hybridizing or binding of the nucleic acids to the capture molecules, preferably a temperature of about 85°C to 98°C.

In yet another preferred embodiment of the present invention the herein above described method additionally comprises as step (e) an activity selected from the group comprising:
(1) amplifying of (a) target molecule(s) bound to (a) capture molecule(s) in said reaction zone;
(2) labeling of (a) target molecule(s) bound to (a) capture molecule(s) in said reaction zone;
(3) ligating of (a) target molecule(s) bound to (a) capture molecule(s) in said reaction zone to another nucleic acid;
(4) performing single base extension with (a) target molecule(s) bound to (a) capture molecule(s) in said reaction zone; and
(5) sequencing of (a) target molecule(s) bound to (a) capture molecule(s) in said reaction zone to another nucleic acid.

In another aspect the present invention relates to the use of a device as defined herein above for efficiently binding a target molecule(s) on a microarray, for enriching (a) target molecule(s), or for specifically selecting (a) target molecule(s), preferably for quantitative and/or multiplexed detection of (a) DNA or RNA molecule(s), or for expression analysis, comparative genomic hybridization, the detection of single nucleotide polymorphisms, or for microarray-based genomic selection-based sequencing.

### BRIEF DESCRIPTION OF THE DRAWINGS

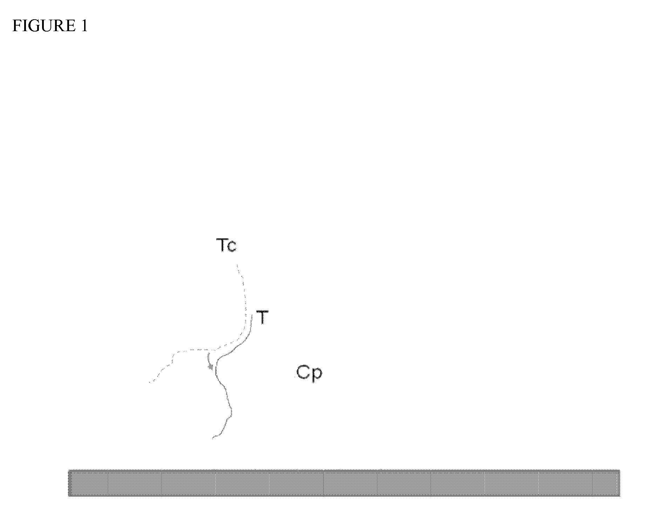

- Fig. 1: depicts a situation in which a single stranded nucleic acid (T) bound to a capture molecule (Cp) is displaced by a complementary single stranded nucleic acid (Tc) by a zipping reaction.

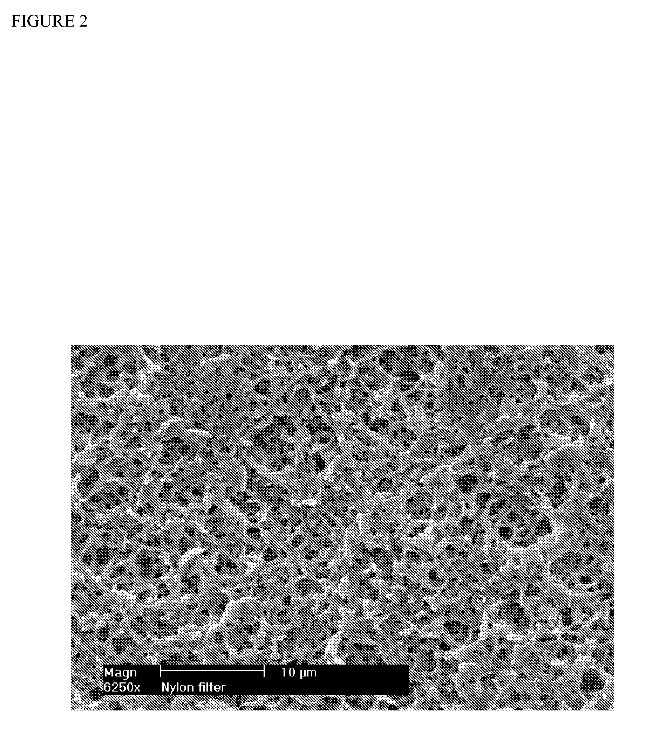

- Fig. 2: shows a nylon membrane which can be used for capturing nucleic acids in a flow through setup by immobilized capture probes on the surface of the membrane.
- Fig. 3: depicts a device according to an embodiment of the present invention in top view and as cross-section. Heating elements underneath the substrate are indicated below with temperatures as example. Reference (1) shows substrate, reference (2) depicts the capture array, reference (3) shows a membrane for nucleic acid strand A, reference (4) shows a membrane for capturing a counter-strand of the nucleic acid, reference (5) shows a sample port, fluid actuation and channel for closed loop circulation.
- Fig. 4: depicts a device according to an embodiment of the present invention in top view and as cross-section similar to the device as shown in Fig. 3, however with the temperature settings of the membranes reversed.
- Fig. 5: shows the effect of storage time of the sample between denaturing and injection in microarray on the density of captured target molecules on the spots of the array.

### DETAILED DESCRIPTION OF EMBODIMENTS

The inventors have developed means and methods, which allow an efficient capture of target nucleic acid molecules on a microarray, in particular by a device for the efficient binding of nucleic acids on a microarray.

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular apparatus, methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As has been set out above, the present invention concerns in one aspect a device for the efficient binding of nucleic acids on a microarray, comprising:
(a) at least one reaction zone comprising a microarray, wherein the microarray comprises a substrate, on which one or more species of capture molecules are immobilized, and
(b) at least one capture zone comprising a porous membrane substrate, on which one or more species of capture molecules are immobilized, which is in fluid connection with the reaction zone;
wherein said capture zone is capable of specifically capturing (a) sense strand(s) or part of (a) sense strand (s) of (a) target molecule(s), whereas (a) complementary antisense strand(s) or part of (an) antisense strand (s) of said target molecule(s) can be captured in said reaction zone; or wherein said capture zone is capable of specifically capturing (an) antisense strand(s) or part of (an) antisense strand (s) of said target molecule(s), whereas (a) complementary sense strand(s) or part of (a) sense strand (s) of said target molecule(s) can be captured in said reaction zone.

The term "device" as used herein refers to a structure, e.g. a receptacle, chamber or container or assembly of such structures, or an instrument, which allows or is suitable for the performance of molecular reactions or interactions, in particular for binding or capturing interactions as well as subsequent, related or associated molecular reactions. The device may correspondingly be equipped with one or more inlet and/or outlet elements, it may comprise one or more surfaces, e.g. reactive surfaces or surfaces with specific functionality, it may comprise one or more functional sectors, for example a reaction or binding sector, a washing sector, a mixing sector, a waiting sector, a measurement sector, a waste sector, a reservoir sector, or any sub-portion or combination thereof. It may further comprise connections between these elements, e.g. tubes or joints; and/or it may comprise reservoirs and repositories for liquids, fluids, chemicals, ingredients, samples or any other entity to be used within the device.

A device according to the present invention comprises a reaction zone. Such a "reaction zone" may be a closed entity comprising only inlet and/or outlet structures being of a minor size in comparison to the size of the zone, or it may be an open structure being in free connection with further entities present in a device.

The reaction zone typically comprises a microarray. The term "microarray" as used herein refers to an ordered or random array presented for interaction between capture molecules in the array and potential interactors in the surrounding environment, e.g. a medium, a reaction solution, preferably a hybridization solution. A microarray may include any two- or three-dimensional arrangement of addressable regions, preferably a two-dimensional arrangement. A typical microarray may contain multiple spots, features, areas of individual immobilization or areas of individual molecular identity. For example, an array may contain more than 2, 5, 10, 50, 100, 500, 750, 1000, 1500, 3000, 5000, 10.000, 20.000, 40.000, 50.000, 70.000, 100.000, 200.000, 300.000, 400.000, 500.000, 750.000, 800.000, 1.000.000, 1.200.000, 1.500.000, 1.750.000, 2.000.000 or 2.100.000 spots, features, capture molecules or areas of individual immobilization or areas of individual molecular identity. These areas may be comprised in an area of less than about 20 cm², less than about 10 cm², less than about 5 cm², less than about 1 cm², less than about 1 mm², less than about 100 µm². In further embodiments the spot size within an microarray may be in a range of about t 1 to 300 µm, e.g. have a size of 10, 50, 100, 150, 200, or 250 µm, or have any suitable value in between the mentioned values. In further embodiments, the probe densities may vary according to necessities. Examples of probe densities are densities between 1000 and 100,000 probes per square µm, e.g. probe densities of 2000, 10,000, 50,000 or 75,000 probes per square µm.

In specific embodiments such spots, features, or areas of individual immobilization may comprise any suitable coverage, e.g. a coverage of 1, 1.5, 2, 3, 4, 5, 6, 7, 8, 9, 10 or more of one or more specific sequences, or genomes or parts of genomes. Thus, a microarray may, for example, comprise 1, 2, 3, 4, 5, 6, 7, 8, 9, 10 or more copies of the same capture molecule. Alternatively or additionally, it may comprise variations of a capture molecule, e.g. mismatch variants, variations with single or multiple nucleotide polymorphisms, different versions of single or multiple nucleotide polymorphisms, capture molecules with different starting or termination sequences, e.g. tags, primer bindings sites, endonuclease recognition sites, however with identical core portions etc.

The term "medium" as used herein refers to any liquid suitable for an interaction between a capture molecule and a target molecule according to the present invention, i.e. as define herein below. Preferably, a medium is a solution or liquid allowing duplexing or hybridizing of a nucleic acid to a particular further nucleic acid, .e.g. a capture probe, under stringent conditions. For example, a medium may comprise, or essentially consist of, or consist of a buffer comprising 50% formamide, 5xSSC, and 1 % SDS, or a buffer comprising 5xSSC and 1% SDS, or a buffer of 40% formamide, 1 M NaCI, and 1 % SDS, or a solution of 0.5 M NaHPO, 4,7% SDS, and 1 mM EDTA, or a buffer of 3xSSC (450 mM sodium chloride/45 mM sodium citrate), or a solution containing 30% formamide, 1 M NaCI, 0.5% sodium sarcosine, and 50 mM MES, pH 6.5. In specific embodiments, a medium as used herein may also comprise ingredients necessary for washing a reaction zone or capture zone. For example, such a medium may have a salt concentration of about 0.02 molar at pH 7; or a salt concentration of about 0.15 M NaCl; or a salt concentration of about 0.2xSSC; or a salt concentration of about 2xSSC containing 0.1% SDS, or a salt concentration 0.1xSSC containing 0.1% SDS. Alternatively, a washing medium may comprise 0.1xSSC/0.1% SDS or 0.2xSSC/0.1% SDS. Furthermore, any suitable commercially available hybridization and/or washing and/or incubation buffer etc. may be used as medium within the device according to present invention.

A medium to be used within a device according to the present invention may further comprise specific salts, e.g. salts of carboxyl groups, or acid addition salts of amino groups of molecules. Salts of a carboxyl group may be formed by methods known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids such as, for example, hydrochloric acid or sulfuric acid, and salts with organic acids such as, for example, acetic acid or oxalic acid.

The microarray typically comprises a substrate, on which one or more species of capture molecules are immobilized. The term "substrate" as used herein refers to any suitable substrate known to the person skilled in the art. The substrate may have any suitable form or format, e.g. it may be flat, curved, e.g. convexly or concavely curved towards, it may be curled or comprise a wavelike format. It may also be organized in round shape structures, in the form of bead-like elements, or beads, or microcarriers, which may, for example, be arranged in an array. The beads may overly a substrate ground or be fixed in the reaction area by connector elements like rods etc. An array may further be comprised of magnetic particles comprising capture molecules.

Typically, the substrate of the reaction zone is a solid support, i.e. comprising support material, which is mainly of non-liquid consistence and thereby allows for an accurate and traceable positioning of the capture molecule on the support material. An example of such a substrate is a solid material or a substrate comprising functional chemical groups, e.g. amine groups or amine-functionalized groups. A corresponding support material or substrate may be, for example, a non-porous substrate. Preferred among non-porous substrates are glass, silicone, fused silica, coated with a silane layer with functionalized groups, like, polyethyleneglycol, amine, epoxide, isothiocyanate, etc., poly-L-lysine coated material, nitrocellulose, polystyrene, cyclic olefin copolymers (COCs), cyclic olefin polymers (COPs), polypropylene, polyethylene and polycarbonate. For example, glass or polystyrene may be used. In addition to a coating or surface treatment with poly-L-lysine, bulk material, in particular glass, may be treated by silanation, e.g. with epoxy-silane or amino-silane or by silynation or by a treatment with polyacrylamide. Particularly preferred is the employment of silanes.

A device according to the present invention comprises in addition to a reaction zone as defined herein a capture zone. Such a "capture zone" may be a closed entity comprising only inlet and/or outlet structures being of a minor size in comparison to the size of the zone, or it may be an open structure being in free connection with further entities present in a device, preferably with a reaction zone as described herein.

The capture zone typically comprises a porous membrane substrate, on which one or more species of capture molecules are immobilized. The term "porous membrane substrate" as used herein refers to a solid, flexible membrane support, i.e. comprising support material which is mainly of non-liquid, flexible consistence and which comprises a significant or substantial proportion of pores, cavities or 3-dimensional irregularities. Porous substrates typically have a strongly increased surface in comparison to non-porous substrates. The present invention envisages porous membrane substrates of any suitable form, thickness, or comprising pores of any suitable size. For instance the porous membrane substrate may be flat, curved, e.g. convexly or concavely curved, it may be curled or comprise a wavelike format. It may also be organized in round shape structures, in the form of bead-like elements, which may, for example, be arranged in an array. The beads may overly a substrate ground or be fixed in the reaction area by connector elements like rods etc. Preferred are membranes having a thickness of about 100 to 1000 µm, more preferably of about 50 to 150 µm. Within the context of the present invention, i.e. for efficient capture and in order to increase the concentration or amount of capture molecules, it is preferred to use a membrane, which comprises a high specific surface. Accordingly, it is preferred to use membranes having small pores. In specific embodiments, the pore size is between 0.1 and 10 µm, more preferably between 0.5 to 5 µm.

In a particularly preferred embodiment of the present invention, a porous membrane substrate is a fabric or felt. In a further particularly preferred embodiment, a porous membrane substrate comprises a random porous structure. Alternatively, it may comprise a regular porous structure. Also envisaged is the presence of 3-dimensional pore structures and/or 2-dimensional pore structures or a mixture of both.

A particularly preferred porous membrane substrate is nylon. Examples of suitable nylon membranes include, but are not limited to, Nytran N^{®}, Nytran SPC^{®} and Biodyne C^{®}.

Techniques for the production of such membranes are known to the person skilled in the art. Preferred examples are the production of anisotropically etched Al-oxide, or the generation of electro-spun fibers. Accordingly, the obtained products, i.e. anisotropically etched Al-oxide or electro-spun fibers may be used as porous membrane substrate within the context of the present invention.

In a further specific embodiment of the present invention a porous membrane substrate may be created in situ. An example of such an in situ creation is a polymerization induced phase separation with a polymer - monomer mixture (e.g. acrylate-PMMA), etc., or thermally induced phase separation of a polymer - solvent mixture.

Capture zone and reaction zone may be present within a device according to the present invention singularly, or either the capture zone or the reaction zone or both may be present within a device according to the present invention more than once. For example, a device according to the present invention may comprise one capture zone and one reaction zone, or it may comprise two capture zones and one reaction zone, or it may comprise two capture zones and two reaction zones, or it may comprise 1, 2, 3, 4, 5, 6, 7, 8, 9, 10 or more capture zones and 1, 2, 3, 4, 5, 6, 7, 8, 9, 10 or more reaction zones etc. Capture zone(s) and reaction zone(s) may further be arranged within a device of the present invention according to any suitable scheme. For example, they may be in fluid connection with each other, e.g. the only reaction zone of the device may be in fluid connection with the only capture zone of the device. Alternatively, a device may comprise more than one capture zone and more than one reaction zone, wherein at least one capture and one reaction zone are in fluid connection. However, a further capture zone and a further reaction zone may not be in fluid connection with said one capture zone and said one reaction zone. For example, a device may comprise a dual, triple or multifold setup of a combination of one capture zone and one reaction zone, which are in fluid connection with each other, but are not in fluid connection with a further set of a capture zone and a reaction zone. In further embodiments, these zones may be integrated in the form of a microfluidic device as defined herein.

The term "fluid connection" as used herein refers to the presence of a connection between the mentioned elements or zones allowing the flow and/or exchange of a liquid, e.g. of hybridization solution, or any other suitable medium between said elements or zones. The fluid connection may be a direct connection, e.g. a juxtaposition between the zones, or include the presence of tubes, joints or conjunctions allowing such a functionality. These structural elements may have any suitable length, diameter, coating, or structure. Examples and further details would be known to the person skilled in the art. In addition, a transportation means as define herein may be present in order to allow for a transport or movement of fluids or liquids.

The present invention also envisages independent, separable and/or removable reaction and/or capture zones within a device. For example, a device may comprise a connection between a capture zone and a reaction zone and/or further possible zones or elements within a device, which can be disassembled allowing the removal of either the reaction zone, or the capture zone or of both or all zones or elements of a device. This option further or alternatively includes the presence of suitable sealing facilities at the connection point(s), the presence of suitable mechanical conjunction elements etc. In certain embodiments, a capture zone may be replaced by a further or different capture zone, or a reaction zone may be replaced by a further or different reaction zone. Such removed elements or zones of the device may, for example, be used in different devices, or in different contexts, e.g. for subsequent analysis steps of bound elements etc. Accordingly, devices of the present invention may be recyclable or reusable after an initial employment, e.g. by the replacement of one or more zones or elements as described herein. Furthermore, for efficient operation it is preferred to use small volumes.

A "capture molecule" as used herein may be any suitable molecule, which allows a specific interaction with a target molecule, in particular a nucleic acid target molecule, form the environment or the medium in the device according to the present invention as defined herein above. Examples of capture molecules which are capable of selecting target molecules form the environment, medium or reaction solution in the device are nucleic acids, proteins, peptides, ligands of any form and format, antibodies, organic or inorganic structures, e.g. carbohydrates or sugars, polymers, or any derivative, modification, analog or combination of the aforementioned. Particularly preferred are capture molecules being nucleic acids.

The term "nucleic acid" as used herein refers to any nucleic acid known to the person skilled in the art, preferably to DNA, RNA, PNA, CNA, HNA, LNA or ANA. The DNA may be in the form of, e.g. A-DNA, B-DNA or Z-DNA. The RNA may be in the form of, e.g. p-RNA, i.e. pyranosysl-RNA or structurally modified forms like hairpin RNA or a stem-loop RNA. The term "PNA" relates to a peptide nucleic acid, i.e. an artificially synthesized polymer similar to DNA or RNA which is used in biological research and medical treatments, but which is not known to occur naturally. The PNA backbone is typically composed of repeating N-(2-aminoethyl)-glycine units linked by peptide bonds. The various purine and pyrimidine bases are linked to the backbone by methylene carbonyl bonds. PNAs are generally depicted like peptides, with the N-terminus at the first (left) position and the C-terminus at the right. The term "CNA" relates to an aminocyclohexylethane acid nucleic acid. Furthermore, the term relates to a cyclopentane nucleic acid, i.e. a nucleic acid molecule comprising for example 2'-deoxycarbaguanosine. The term "HNA" relates to hexitol nucleic acids, i.e. DNA analogues which are built up from standard nucleobases and a phosphorylated 1, 5-anhydrohexitol backbone. The term "LNA" relates to locked nucleic acids. Typically, a locked nucleic acid is a modified and thus inaccessible RNA nucleotide. The ribose moiety of an LNA nucleotide may be modified with an extra bridge connecting the 2' and 4' carbons. Such a bridge locks the ribose in a 3'-endo structural conformation. The locked ribose conformation enhances base stacking and backbone pre-organization. This may significantly increase the thermal stability, i.e. melting temperature of the oligonucleotide. The term "ANA" relates to arabinoic nucleic acids or derivatives thereof. A preferred ANA derivative in the context of the present invention is a 2'-deoxy-2'-fluoro-beta-D-arabinonucleoside (2'F-ANA). Nucleic acid molecules may comprise a combination of any one of DNA, RNA, PNA, CNA, HNA, LNA and ANA, e.g. mixtures of LNA nucleotides with DNA or RNA bases. Nucleic acid molecules as defined herein above may be in the form of short oligonucleotides, long oligonucleotides or polynucleotides.

In certain embodiments of the present invention, a nucleic acid may be present in a duplex form, i.e. comprising a strand and a complementary or anti-parallel counter-strand of a nucleic acid molecule associated by base pairing between the strands, or a sense or antisense-strand of a nucleic acid molecule. The term "sense strand" as used herein refers to a molecule comprising the sequence, which is the same as that of a messenger RNA copy that is or may be translated into a protein. The term, within the context of the present invention, additionally refers to molecules comprising one strand of a duplex nucleic acid molecule, which is not identical to its complementary counter-strand, irrespective of the presence of a messenger RNA copy or its expression into a protein. A sense strand according to the present invention may thus be defined in accordance with the definition of genetic units, e.g. genes for which a derived transcript or protein sequence is known, or a sense strand may be defined according to genomic sequence information as strand 1 or strand A of a genomic sequence, which is not identical to its counter-strand (strand 2 or strand B). For genomic sequences, the exact identity of the sense strand, i.e. the question of its identity to transcribed mRNA sequences, may be irrelevant as long as the sense strand is understood as the complementary sequence of the antisense strand, and vice versa. For nucleic acid fragments or sub-genomic portions of a genome, for which no functional information or information on genetic units or expression units is available, a sense strand of a duplex nucleic acid molecule may be any of the two strands, the antisense strand being the opposite or complementary strand thereof. Accordingly, the term "anti-sense strand" as used herein refers to the molecule comprising the complementary or opposite strand with respect to the sense strand as defined above.

The term "complementary" as used herein refers to the presence of matching base pairs in opposite nucleic acid strands. For example, to a nucleotide or base A in a sense strand a complementary or antisense strand binds with a nucleotide or base T, or vice versa; likewise to a nucleotide or base G in a sense strand the complementary or antisense strand binds with a nucleotide or base C, or vice versa. This scheme of complete or perfect complementarity may, in certain embodiments of the invention, be modified by the possibility of the presence of single or multiple non-complementary bases or stretches of nucleotides within the sense and/or antisense strand(s). Thus, to fall within the notion of a pair of sense and antisense strands, both strands may be completely complementary or may be only partially complementary, e.g. show a complementarity of about 60%, 70%, 75%, 80%, 85%, 90%, 91%, 92%, 93%, 94%, 95%, 96%, 97%, 98%, 99%, 99.5%, or 100% between all nucleotides of both strands. Non-complementary bases may comprise one of the nucleotides A, T, G, C, i.e. show a mismatch e.g. between A and G, or T and C, or may comprise any modified nucleoside bases including, for example, modified bases as described in WIPO Standard ST.25 (1998), Appendix 2, Table 2. Furthermore, the present invention also envisages complementarity between non-identical nucleic acid molecules, e.g. between a DNA strand and a RNA strand, a DNA strand and a PNA strand, a DNA strand and a CNA strand, etc., or a complementarity between a nucleic acid molecule and an organic polymer.

In other embodiments, a nucleic acid molecule may be present in a single stranded form. The term "single stranded form" as used herein means that the nucleic acid molecule shows no base pairing between two nucleic acid molecules. A single stranded form of a nucleic acid may be obtained by melting or denaturation of duplex nucleic acid molecules, e.g. by heating such molecules to temperatures of about 85°C to 98°C, preferably to 95°C. Temperature conditions may further be made dependent on the sequence of the nucleic acid, its length, or medium properties, such as the presence of salt, detergents etc. Alternatively, single stranded forms of a nucleic acid may be obtained by a de novo synthesis of nucleic acid molecules, e.g. according to suitable synthesis methods known to the person skilled in the art, or by suitable filtering or enrichment process, including the methods as envisaged by the present invention.

The term "peptide" in the context of the present invention refers to any type of amino acid sequence comprising more than 1 amino acids, in particular short sequences of up to 40 or 50 amino acids length. The term, in certain embodiments, also refers protein structures or functional derivatives thereof. Furthermore, the peptide may be combined with further chemical moieties or functionalities. The term "polypeptide" as used herein refers medium or long amino acid sequences, preferably comprising 40 or 50 amino acids. The term, in certain embodiments, also refers protein structures or functional derivatives thereof. Furthermore, the polypeptide may be combined with further chemical moieties or functionalities. The term "carbohydrate" as used herein refers to an organic compound with the empirical formula C*ₘ*(H₂O)*ₙ*; that is, consists only of carbon, hydrogen, and oxygen, with a hydrogen:oxygen atom ratio of 2:1. The group comprises monosaccharides, disaccharides, oligosaccharides, and polysaccharides. Reference is preferably made to sugars, i.e. to monosaccharides and disaccharides. A "modification" of the polypeptide, protein or peptide according to the present invention may be an acetylation, pegylation, hesylation, formylation, phosphorylation, amidation, derivatization by known protecting/blocking groups, specific chemical cleavage, proteolytic cleavage, a linkage to a cellular ligand or other protein or hapylation, i.e. a fusion with a glycine-rich homo-amino-acid polymer (HAP), etc. Such modifications may be carried out by suitable techniques known to the person skilled in the art. Additionally, the polypeptide, peptide or variant may contain one or more non-classical amino acids.

A capture zone according to the present invention comprising one or more species of capture molecules as defined herein is typically capable of specifically capturing or binding to a target molecule, in particular a single stranded target molecule, i.e. a sense or antisense strand of a nucleic acid molecule as defined herein above. Accordingly, a reaction zone according to the present invention comprising one or more species of capture molecules as defined herein is typically capable of specifically capturing or binding a target molecule, in particular a single strand, i.e. a sense or antisense strand of a nucleic acid molecule as defined herein above. Thus, in typical embodiments of the present invention the mentioned capture zone may, within the context of a device of the invention, be able to capture one strand of a duplex nucleic acid target molecule, whereas the mentioned reaction zone is able to capture the corresponding counter-strand of said duplex nucleic acid target molecule. For example, if a capture zone is capable of specifically capturing or binding a sense strand of a nucleic acid target molecule, as defined herein above, the corresponding reaction zone is capable of specifically capturing or binding an antisense strand of said nucleic acid target molecule. Alternatively, if a capture zone is capable of specifically capturing an antisense strand of a nucleic acid target molecule, as defined herein above, the corresponding reaction zone is capable of specifically capturing a sense strand of said nucleic acid target molecule.

In further embodiments of the present invention, a capture zone according to the present invention comprising one or more species of capture molecules as defined herein is typically capable of specifically capturing or binding to a part of a target molecule, in particular a part of a single stranded target molecule, i.e. a part of a sense or antisense strand of a nucleic acid molecule as defined herein above. Accordingly, a reaction zone according to the present invention comprising one or more species of capture molecules as defined herein is typically capable of specifically capturing or binding to a part of a target molecule, in particular a part of a single stranded target molecule, i.e. a part of a sense or antisense strand of a nucleic acid molecule as defined herein above. The terms "part of a target molecule", "part of a single stranded target molecule" and "part of a sense or antisense strand of a nucleic acid molecule" as used herein refer to situations, in which not the entire target molecule, typically the entire single stranded molecule, binds to or is complementary to a capture molecule, but wherein only a portion thereof is complementary and/or able to bind. This part or portion of said target molecule may have a minimal length of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 nucleotides. Furthermore, a capture molecule binding only to a part of a target molecule may have a minimal length of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 nucleotides. In further additional or alternative embodiments, both interaction molecules, i.e. a capture molecule as defined above and a target molecule as defined above may, over their entire length, show a complementarity of about 30%, 33%, 35%, 40%, 45%, 50%, 55%, 60%, 70%, 75%, 80%, 85%, 90%, 91%, 92%, 93%, 94%, 95%, 96%, 97%, 98%, 99% or 99.5%. Non-complementary regions or portions may include linker sequences, artificial primer binding sites, artificial endonuclease recognition sites, tags, bar-coded sequences for identification purposes etc. The presence of such non-complementary sections may be considered by an adjustment of binding parameters, e.g. the temperature, the concentration of hybridization solution ingredients, the flow velocity or rate in the device, the interaction time etc. Such parameters and their setup would be known to the person skilled in the art. Also envisaged is a mutual exclusive mixture of binding capabilities for the sense and antisense strands in cases in which the device comprises at least two capture zones and/or at least two reaction zones.

Typically, the capture molecules, in particular nucleic acids, are immobilized on the substrate as described herein above, e.g. in the form of a microarray, or on a porous membrane substrate as defined herein. The term "immobilized" as used herein refers to the association of one or more capture molecules to a supportive substrate via molecular interactions which position the molecule at a specific area of the substrate and concomitantly impede a detaching of the capture molecule, e.g. during washing, rinsing or interaction steps etc. Typically, such molecular interactions are based on covalent chemical bonds between structural elements or functional groups of the support material and the capture molecule to be immobilized, e.g. corresponding functional groups of nucleic acids, as known to the person skilled in the art. The immobilization may, for example, be carried out by crosslinking the capture molecules by heat or light, i.e. by forming molecular interactions or bonds that link both structural elements together under the influence or driven by the energy provided by an energy source like heat or light, or via a chemical immobilization.

The term "size of the capture molecule" within the context of adsorption interactions relates to the number of binding sites that are present. Although any one binding site may, in principle, dissociate from the surface of the substrate at any time, the effect of a large number of binding sites is that the capture molecule as a whole will remain bound. By applying energy in the form of heat, e.g. at a temperature of about 40 to 150°C, preferably 50 to 120°C, more preferably 60 to 110°C, the physical adsorption of the capture molecule to the support material may be enhanced and the time necessary for an efficient immobilization may be shortened. The crosslinking by heat may be carried out for any suitable period of time known to the person skilled in the art, e.g. 2 min to 12 hours, preferably 10 min to 8 hours, more preferably 30 min to 6 hours, even more preferably 45 min to 4 hours even more preferably 1 hour to 3 hours and most preferably for 2 hours. The crosslinking by heat or baking may be carried out by any suitable means known to the person skilled in the art, for example a drying chamber or an oven. In addition to the temperature, also other parameters like humidity, aeration or ventilation may be adjusted to suitable values known to the person skilled in the art. The crosslinking by heat or baking may also be combined with other forms of immobilization like crosslinking by light or chemical immobilization.

Crosslinking by light is typically performed by applying light of a typical wavelength, e.g. in a range of 150 to 550 nm, preferably in a range of 200 to 500 nm to capture molecules in order to induce an interaction between the capture molecules and support material. Typically, the induced interaction between the capture molecules and the support material is a covalent binding of the nucleic acid to the material. Crosslinking by light may, for example, be carried out by using UV light. UV crosslinking is one of the simplest ways to ensure covalent binding of a support material to a probe. In the case of nucleic acids, the linkage proceeds through the bases of a nucleic acid molecule, e.g. thymine, guanine, adenine, cytosine or uracil residues, which react with corresponding and suitable functional chemical groups on the support material, as known to the person skilled in the art. The presence and number of functional chemical groups on or inside the support material may be controlled and adjusted via suitable chemical activation processes. Such activation processes may, for instance, provide specifically localized functional groups on or within a support material and facilitate a specific interaction between the capture molecules and the material within the context of these localized functional groups. The presence and number of functional group on or inside the support material may also have an influence on the orientation and freedom of the immobilized capture molecules. For example, the presence of a higher number of functional groups may lead to an immobilization at different points within the capture molecule. Furthermore, the presence of corresponding reactive elements within the capture molecule may be used for a control of the orientation of the capture molecule on the support material.

A "chemical immobilization" as mentioned herein may be an interaction between the support material and the capture molecule based on chemical reactions. Such a chemical reaction does typically not rely on the input of energy via heat or light, but can be enhanced by either applying heat, e.g. a certain optimal temperature for a chemical reaction, or light of certain wavelength. For example, a chemical immobilization may take place between functional groups on a support material and corresponding functional elements on the capture molecules. Such corresponding functional elements in the capture molecules may either be as part of the chemical inventory of a molecule, or be additionally introduced. An example of such a functional group is an amine group. Typically, the capture molecule to be immobilized, e.g. a nucleic acid, comprises a functional amine group or is chemically modified in order to comprise a functional amine group. Means and methods for such a chemical modification are known to the person skilled in the art and can, for example, be derived from organic chemistry textbooks like Organische Chemie by Hart et al., 2007, Wiley-Vch or Organische Chemie by Vollhardt et al., 2005, Wiley-Vch. The localization of said functional group within the capture molecule to be immobilized may be used in order to control and shape the binding behavior and/or orientation of the capture molecule, e.g. the functional group may be placed at the end or tail region of the capture molecule or in the centre of the capture molecule. A typical reaction partner for a capture molecule to be immobilized comprises moieties which are capable of binding to such capture molecules, e.g. to nucleic acids such as amine-functionalized nucleic acids. Examples of such support material are aldehyde, epoxy or NHS substrates. Such material is known to the person skilled in the art. Functional groups, which impart a connecting reaction between capture molecules, which are chemically reactive by the introduction of an amine group, and a support material are known to the person skilled in the art. An alternative reaction partner for capture molecules to be immobilized may have to be chemically activated, e.g. by the activation of functional groups, available on the support material. The term "activated support material" relates to a material in which interacting or reactive chemical functional groups were established or enabled by chemical modification procedures as known to the person skilled in the art. For example, a substrate comprising carboxyl groups has to be activated before use. Furthermore, there are substrates available that contain functional groups that can react with specific moieties already present in the nucleic acids. Some of these reactions are enhanced by heat or UV. An example are amine groups on the surface of the substrate, which can be bound to specific bases in the DNA.

Alternatively, the capture probes may be synthesized in situ or directly on the substrate. Suitable methods for such an approach are known to the person skilled in the art. Examples are manufacture techniques developed by Agilent Inc., Affymetrix Inc., Nimblegen Inc. or Flexgen BV. Typically, lasers and a set of mirrors that specifically activate the spots where nucleotide additions are to take place are used. Such an approach may provide, for example, spot sizes of around 30 µm or larger. Capture probes may accordingly have a length of up to about 80 nucleotides. In a different, also envisaged technique, the capture probes may be deposited by using a non-contact inkjet printing process, in which oligonucleotide monomers are deposited uniformly onto specially-prepared glass slides. This in situ synthesis process may typically produce 60-mer length oligonucleotide probes, base-by-base, e.g. from digital sequence files.

For substrates in a reaction zone comprising a microarray as defined herein preferably an immobilization technique based on in situ synthesis or a chemical immobilization may preferably be used. For porous membrane substrates in capture zones as defined herein a chemical immobilization technique or an immobilization via crosslinking by heat, or light, in particular UV may preferably be used.

The herein mentioned reaction zone and/or capture zone may comprise "one or more species" of a capture molecule, i.e. one or more different types of molecule may be present in a microarray or on a porous membrane substrate such as nucleic acids and proteins, nucleic acids and carbohydrates etc. Alternatively, the term "one or more species" also relates to capture molecules of the same category or having the same form or format, e.g. nucleic acids, but which are not identical or similar in their molecular identity, e.g. the sequence in the case of nucleic acids. Thus, a reaction zone or a capture zone according to the present invention may comprise different nucleic acids, or different proteins, or different carbohydrates, or different antibodies, or different ligands etc., or any combination of different nucleic acids and different proteins etc., preferably different nucleic acids. If capture molecules of a different molecular identity, in particular nucleic acids, are present in a reaction zone or a capture zone according to the present invention, these capture molecules may be partially identical or partially similar, i.e. have, in particular in the case of nucleic acids, overlaps in terms of sequence or may have no overlap. Nucleic acid capture molecules according to the present invention may, for example, comprise, cover or represent the sequence of any suitable area or percentage of a genome, e.g. between about 0.00001 % to about 30 % of a genome, such as at least about 0.00001, 0.00005, 0.0001, 0.0005, 0.001, 0,005, 0.01, 0.02, 0.05, 0.06, 0.07, 0.08, 0.09, 0.1, 0.11, 0.12, 0.13, 0.14, 0.15, 0.16, 0.2, 0.3, 0.4, 0.5, 0.75, 0.8, 0.9, 1, 1.5, 2, 3, 4, 5, 10, 15, 20, or 30 % of the genome of an organism, preferably of a mammal genome, more preferably of the human genome and/or be complementary to such regions. Such an area or percentage may comprise, for example, a group of about 2 to 5.000 genes, e.g. 2, 3, 4, 5, 6, 7, 8, 9, 10, 15, 20, 25, 30, 50, 100, 150, 200, 350, 500, 750, 1000, 1200, 1500, 2000, 2500, 3000, 4000, 5000 or more than 5000 genes. Such genes may either be localized in adjacent genomic areas o regions or my alternatively be dispersed throughout the genome. Also sub-groupings, combinations, pattern, e.g. pattern derived from expression data etc. of genes are envisaged.

A "capturing" as mentioned herein refers to the interaction between capture molecules of the device according to the invention and nucleic acid target molecules, e.g. molecules present in the medium or sample solution or provided within hybridizing solutions etc. Such an interaction may be any suitable molecular, sub-molecular or macro-molecular interaction known to the person skilled in the art, e.g. an affinity interaction, an interaction based on van-der-Waals forces, an interaction based on hydrogen-bonding and/or an interaction based on electric charges, e.g. between differently charged molecules. A typical and preferred example of such an interaction is a hybridization reaction involving nucleic acids. Further envisaged is, for example, the interaction of a nucleic acid with a carbohydrate structure. Based on the interaction the interacting partner may be bound to one or more capture molecules of the device according to the present invention and thereby be removed from a surrounding environment, a sample solution, or a hybridization or reaction solution. The capturing of target molecules is preferably specific. The term "specific capturing" as used in the context of a device according to the present invention has different meanings, which largely depend on the identity of the target molecule and/or the interaction type and which follow in general suitable rules and definitions known in the art. For example, a nucleic acid-nucleic acid interaction may be considered to be specific if nucleic acid target molecules may interact which are entirely or partially complementary to each other or which are at least about 30% to 99% complementary to each other over their entire length or a portion of the entire length. An interaction between a nucleic acid and a carbohydrate may be considered to be specific according to known standards in the art.

A "target molecule" as used herein may be any suitable molecule, in particular nucleic acid molecule, which allows a specific interaction between the target molecule and a capture molecule or within a capture zone or reaction zone as described herein. A target molecule may typically be a nucleic acid as defined herein above, e.g. as genomic DNA, cDNA, mRNA or an amplified DNA/RNA products, or a synthetically produced nucleic acid, e.g. an oligonucleotide, or any grouping or combination thereof. Furthermore, the target molecules may be a specific allelic variant of these molecules, e.g. mutant or disease-associated forms, or an allelic variant, which is useful in forensic identifications. The target molecule to be used within the context of the present invention, in particular within the context of the device as defined herein, may be a single type of target molecule (for example a nucleic acid having a specific nucleotide sequence), or it may comprise a class of molecules (for example nucleic acids of varying lengths including or comprising a specific nucleotide sequence, or nucleic acids corresponding to various alleles of a particular gene, or nucleic acids comprising specific sequences and linker sequences or common primer binding site sequences etc.).

Target molecules may be obtained or derived from samples. The present invention accordingly envisages any sample, which include target molecules as defined herein. Such samples may, for example, include samples derived from or comprising stool, whole blood, serum, plasma, tears, saliva, nasal fluid, sputum, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, fluid harvested from a site of an immune response, fluid harvested from a pooled collection site, bronchial lavage, urine, biopsy material, e.g. from all suitable organs, e.g. the lung, the muscle, brain, liver, skin, pancreas, stomach, etc., a nucleated cell sample, a fluid associated with a mucosal surface, hair, or skin. In addition, samples from environmental sources, e.g. water samples, meat or poultry samples, samples from sources of potential contamination etc. may be used.

In some embodiments, target molecules may be directly obtained from samples. In other situations samples may be subjected to sample preparation techniques, e.g. based on standard protocols, including, for example, partial purification, which renders the target molecules more accessible to binding partners.

For example, blood samples may be centrifuged to separate fractions including whole cells or membranes from serum, feces samples may be sectioned and homogenized with physiologically acceptable buffer and detergent, sputum samples may be liquefied and fractionated. Furthermore, antibiotics or bactericides may be added to samples to prevent further growth of any organisms present. Whole cells may also be removed or may be lysed to release their contents.

Preferably, for the preparation of nucleic acid containing samples, inhibitors of DNA and RNA degrading enzymes and proteinases may be added. Furthermore, nucleic acid target molecules may also be amplified prior to detection, e.g. via suitable PCR reactions or ligase chain reactions as would be known to the person skilled in the art.

In further embodiments, nucleic acids in samples may be sheared or cut into smaller fragments (e.g., by mechanical shearing or restriction enzyme digestion). Preferred is a shearing process leading to nucleic acid molecules of a size of 50 to 500 nucleotides, preferably of 200 nucleotides. Typically, a shearing technique by focused ultrasound may be used, e.g. based on any suitable apparatus such as an instrument marketed by Covaris. Heterogeneous samples may further be purified to remove substantially all non-nucleic acid molecules prior to loading the sample into the device. Additionally, or alternatively, heterogeneous samples may be processed by separation techniques such as capillary electrophoresis, e.g. in order to deselect molecules outside of a desired length range, preferably molecules having a size outside of a range of 50 to 500 nucleotides.

In a specific embodiment of the present invention a target molecule, in particular nucleic acid molecule, is provided in a single stranded form, e.g. as sense or antisense strand of a duplex molecules, or as a synthetic single stranded molecules such as a synthetic oligonucleotide. In further embodiments, the target molecule may also be provided in partially single stranded form, e.g. being single stranded in a proportion of 10%, 20%, 30%, 40%, 50%, 60%, 70%, 80%, 90%, 95% or 99% or any value in between. In further embodiments the target molecule may be double stranded. In case the target molecule is provided in a double stranded or partially single stranded form, it may be required to pass the target molecule over or keep it for a certain period of time in a denaturing zone or sector as defined herein. In one embodiment of the invention the target molecule is provided in a denatured form. The term "denatured form" as used herein refers to a single stranded form or an essentially single stranded form, e.g. comprising at least about 75% single stranded molecules, of a nucleic acid target molecule. A single stranded form may, for example, be obtained by a nucleic acid melting or denaturation process. In preferred embodiments said process includes a heating step at temperatures of about 85°C to 98°C, preferably at 95°C, of a sample probe as defined herein, for a suitable amount of time, e.g. for about 5 min, 10 min, 15 min, 20 min or longer any time period between these values. Such a denatured sample may accordingly be provided in the device in order to allow an interaction with capture molecules bound in the capture zone and the reaction zone of the device. In further embodiments of the present invention, a denatured form of a target molecule or of sample may be obtained within the device itself, e.g. in a denaturing zone or sector, which is connected to the capture and/or reaction zone(s) as defined herein. Accordingly, double stranded samples or target molecules may be introduced into said denaturing zone or sector and converted into single stranded or essentially single stranded molecules. In specific embodiments, said denaturing zone may be located within one or more of the reaction or capture zones, or within any other portion of the device.

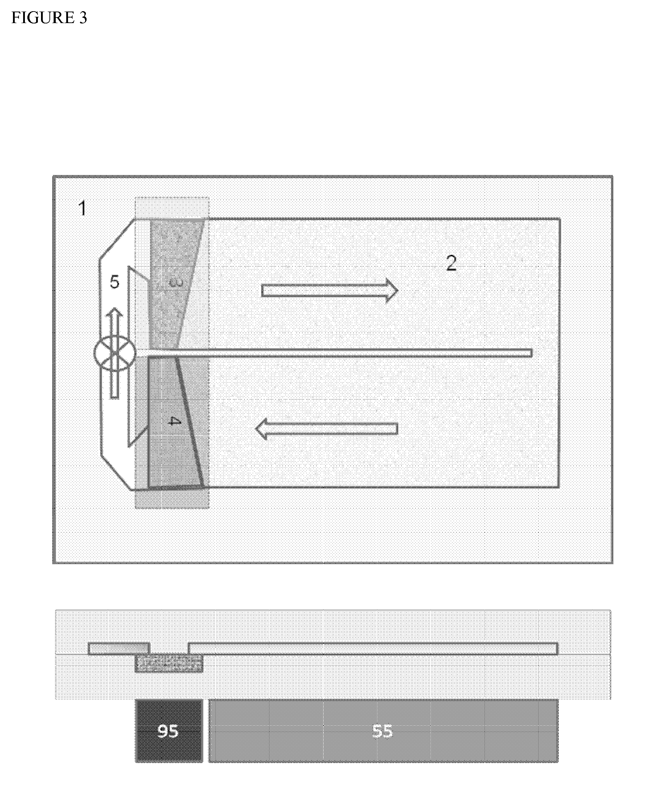

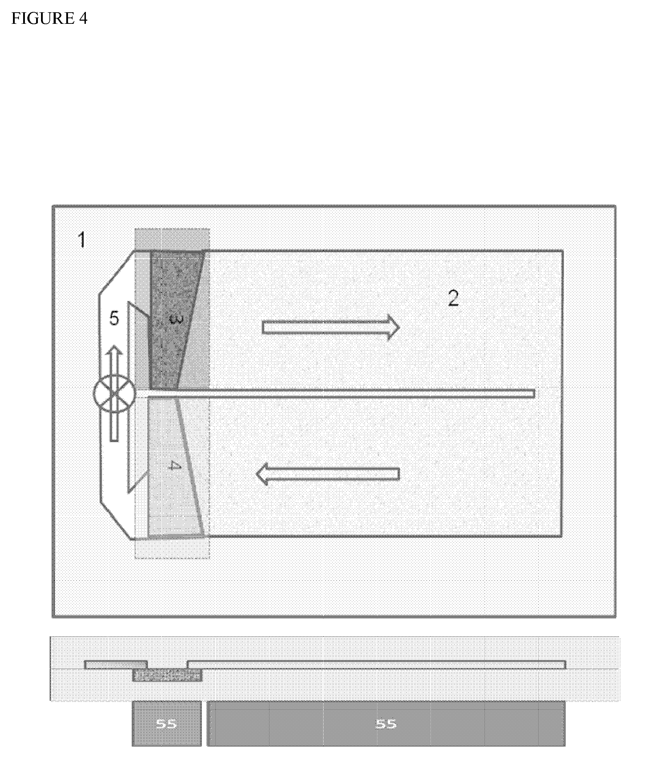

In specific embodiments of the present invention, a device according to the present invention comprises at least two capture zones wherein one capture zone is capable of specifically capturing (a) sense strand(s) or part of (a) sense strand (s) of (a) target molecule(s), and the other capture zone is capable of specifically capturing (a) complementary antisense strand(s) or part of (an) antisense strand (s) of (a) target molecule(s), or vice versa. Also envisaged is a mutual exclusive mixture of binding capabilities for the sense and antisense strands in the at least two capture zones. In these embodiments both strands of a duplex target molecule may be captured in capture zones as defined herein above. These capture zones are preferably arranged such that after the first of these capture zones a reaction zone as defined herein above is placed or located within a liquid or medium flow path within the device. Accordingly, after the binding of the sense strand molecule(s) in the first of said capture zones, the remaining counter-strand may be moved or moves to the reaction zone having capture molecules complementary to the remaining counter-strand. Thereby, the binding of single-stranded counter-strands of the sense strand molecules bound to the first capture zone to the reaction zone may be enhanced. A second capture zone, following the reaction zone may similarly capture said counter-strands, which did not bind to the reaction zone. By subsequently allowing sense strands initially bound to the first capture zone to become available in the medium or environment of the device, a binding of these strands to the reaction zone, which may in specific embodiments comprise capture molecules complementary to the sense strand of the target molecule, may be induced. Further variants of this setup, e.g. the inclusion of a second or further reaction zone, the inclusion one or more further capture zones or the combination with further functionalities etc. are also envisaged by the present invention. In a specific embodiment of the invention, if a reaction zone comprises capture molecules for both strands of a target molecule, one reaction zone may be combined with two capture zones, one of these specifically capturing sense strand target molecules, the other one specifically capturing antisense strand target molecules. A specific detailed version of this preferred device form is depicted in Figs. 3 and 4.

In preferred embodiments of the present invention, the device as defined herein above comprises at least one temperature control and/or regulating unit for controlling and/or regulating the temperature within the device. The term "temperature control and/or regulating unit" as used herein refers to a mechanical, electrical (resistive), radiation, microwave, or any other suitable heating device or element, which is capable of providing and/or keeping a temperature in the range of between about 0.5°C and 120°C, preferably in the range of between about 20°C and 100°C, more preferably in the range of between about 35°C and 95°C. The temperature control and/or regulating unit may, thus, function either as heater, if the environmental temperature in the device or a zone or sector of the device is below a certain predefined value or as cooler if the environmental temperature in the device or a zone or sector of the device is above a certain predefined value. The temperature control and/or regulating unit may accordingly comprise a sensor for measuring the environmental temperature and an element allowing the initiation of cooling or heating activity if the measured temperature is not at the predefined value. A predefined value may be any suitable value, preferably a temperature value of between about 0.5°C and 120°C, preferably in the range of between about 20°C and 100°C, more preferably in the range of between about 35°C and 95°C. A temperature control and/or regulating unit may be either independently addressable, e.g. via its own interface to the use, or be integrated in a network of similar units or be connected to a regulating electronic device etc. The device may preferably comprise between one and 15 temperature control and/or regulating units. If there is more than one such unit, their temperature may be different with respect to the other units or may be identical or similar. By setting such units in the device, or in a zone of the device to different temperatures, for example a temperature gradient within said sector may be generated. Preferably, the reaction zone may be set to a temperature which favors the melting of nucleic acid molecules, or which favors the interaction between target molecules and capture molecules in the capture and reaction zone. In particularly preferred embodiments of the present invention said temperature control and/or regulating unit may be resistive heaters in a metal block, a resistive heater attached to a good thermal conductor, e.g. a metal, or a high conducting glass, or a thin film resistive heater on a glass substrate. Alternatively, it may be a thin film heater integrated in the substrate of the device, e.g. underlying the microarray sector.

In particularly preferred embodiments of the present invention said temperature control and/or regulating unit may be located in a capture zone of the device as defined herein. In such a scheme, a capture zone may be brought to or kept at a temperature which allows an interaction between capture molecules and complementary strands of a target molecule. In such a scheme, single strands of a duplex target molecule may be bound to the capture zone, allowing the complementary strands to pass the capture zone and enter the reaction zone, allowing an interaction between both entities. Alternatively, a capture zone may be brought to or kept at a temperature at which no interaction between capture molecules and complementary strands of a target molecule is possible, thereby releasing previously bound single stranded molecules or allowing said strands of a target molecule to leave the capture zone and enter the reaction zone, allowing an interaction between both entities.

In further preferred embodiments of the present invention said temperature control and/or regulating unit may additionally be located in a reaction zone as defined herein. In such a scheme, in a reaction zone a temperature may be set which allows a quantitative, semi-quantitative or specific binding, or highly specific binding of a target molecule to capture molecules, e.g. in the microarray.

In further embodiments of the present invention said temperature control and/or regulating unit may be located at any other specific point, sector or part of a device according to the present invention. device.

In a further preferred embodiment of the present invention, one of the at least two, e.g. 2, 3, 4, 5, 6 or more capture zones is kept at or brought to a nucleic acid melting temperature or a temperature not allowing hybridizing or binding of nucleic acid(s) target molecules to the capture molecules, whereas one, two, 3, 4, 5, 6 or more other capture zone(s) are kept at or brought to a temperature allowing hybridizing or binding of nucleic acid(s) to the capture molecules. In further specific embodiments of the present invention one of the at least two, e.g. 2, 3, 4, 5, 6 or more capture zones is kept at or brought to a temperature of about 85°C to 98°C, whereas one, two, 3, 4, 5, 6 or more other capture zone(s) are kept at or brought to a temperature of 45°C to 65°C. In specific embodiments, the temperature may be in non-hybridizing sectors of the device at 85°C, 86°C, 87°C, 88°C, 89°C, 90°C, 91°C, 92°C, 93°C, 94°C, 95°C, 96°C, 97°C, 98°C or higher or at any value between the indicated temperature values. In further embodiments, the temperature may be in a hybridizing sector of the device, e.g. in (a) capture zone(s) or reaction zone(s) at 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C or higher, or at any value between the indicated temperature values. In further embodiments the temperature within the device may be modulated according to suitable schemes, e.g. there may be a temperature gradient between (a) capture zone(s) allowing hybridizing or binding of nucleic acid(s) to the capture molecules and (a) capture zone(s) not allowing hybridizing or binding of nucleic acid(s) target molecules to the capture molecules. In further embodiments, temperature gradients between said capture zone(s) and further sectors of the device are envisaged, e.g. the reaction zone, or optional washing, denaturation, reservoir zones or sectors etc. In further embodiments, when more than two capture zones are used, there may be a temperature difference between the capture zone(s) allowing hybridizing or binding of nucleic acid(s) to the capture molecules and/or between the capture zone(s) not allowing hybridizing or binding of nucleic acid(s) target molecules to the capture molecules. The temperature and/or any temperature gradient may be made dependent and be controlled in dependence of the temperature of proximate capture zones, or the molecule to be captured, the length of the molecule to be captured, the amount of molecules to be captured, the complexity of the sample, the presence of mismatches or presumed mismatches between the capture molecule and a target molecule etc.

In preferred embodiments of the present invention a device as described herein above may comprises within the reaction zone different types, amounts, concentrations, or forms of capture molecules. For example, the reaction zone may comprise a capture molecule or capture molecules complementary to the sense strand of the target molecule as defined above and, at the same time, capture molecules complementary to the antisense strand of the target molecule as defined above. In such a scheme, a reaction zone is capable of binding both strands, of a duplex nucleic acid target molecule.

In further embodiments, the reaction zone may comprise a capture molecule or capture molecules complementary to the sense strand of the target molecule as defined above and, at the same time, capture molecules complementary to the antisense strand of the target molecule as defined above. In such a scheme, a reaction zone is capable of binding both strands of a duplex nucleic acid target molecule or essentially both strands of a duplex nucleic acid target molecule, or about 60%, 70%, 80%, 90%, 91%, 92%, 93%, 94%, 95%, 96%, 97%, 98%, or 99%, or 99.5% of both strands of a duplex nucleic acid target molecule.

In further embodiments, the reaction zone may additionally or alternatively comprise a capture molecule or capture molecules complementary to (a) part(s) of a sense strand of the target molecule as defined above and, at the same time, capture molecules complementary to (a) part(s) of the antisense strand of the target molecule as defined above. This part or portion of the capture molecule, which is complementary to said sense strand of the target molecule or said antisense strand of the target molecule target, may have a minimal length of at least 10, 11, 12, 13, 14, 15, 16, 17, 18, 19, 20, 21, 22, 23, 24, 25, 26, 27, 28, 29, 30,31,32,33,34,35,36,37,38,39,40,41,42,43,44,45,46,47,48,49,50,51,52,53,54, 55, 56, 57, 58, 59 or 60 nucleotides. Furthermore, a capture molecule binding only to a part of a sense or antisense strand may have a minimal length of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 nucleotides. In further additional or alternative embodiments, both interaction molecules, i.e. a capture molecule as defined above and a sense or antisense strand of a target molecule as defined above may, over their entire length, show a complementarity of about 30%, 33%, 35%, 40%, 45%,50%, 55%, 60%, 70%, 75%, 80%, 85%, 90%, 91%, 92%, 93%, 94%, 95%, 96%, 97%, 98%, 99% or 99.5% or of any value in between. Non-complementary regions or portions may include linker sequences, artificial primer binding sites, artificial endonuclease recognition sites, tags, bar-coded sequences for identification purposes etc. The presence of such non-complementary section may be considered by an adjustment of binding parameters, e.g. temperature, the concentration of hybridization solution ingredients, flow velocity in the device etc. Such parameters and their setup would be known to the person skilled in the art. In such a scheme a reaction zone may be capable of binding both strands of a duplex nucleic acid target molecule or essentially both strands of a duplex nucleic acid target molecule, or about 50%, 55%, 60%, 65%, 70%, 80%, 90%, 91%, 92%, 93%, 94%, 95%, 96%, 97%, 98%, or 99%, of both strands of a duplex nucleic acid target molecule.

In further embodiments, the reaction zone may comprise a capture molecule or capture molecules complementary to only the sense strand of the target molecule as defined above. Additionally or alternatively, the reaction zone may comprise a capture molecule or capture molecules complementary to (a) part(s) of a sense strand of the target molecule as defined above. In such a scheme a reaction zone is capable of binding the sense strand of a duplex nucleic acid target molecule as defined above or essentially the sense strand of a duplex nucleic acid target molecule, or about 30%, 33%, 35%, 40%, 45%,50%, 55%, 60%, 70%, 80%, 90%, 91%, 92%, 93%, 94%, 95%, 96%, 97%, 98%, or 99%, or 99.5% of the sense of a duplex nucleic acid target molecule.

In further embodiments, the reaction zone may comprise a capture molecule or capture molecules complementary to only the antisense strand of the target molecule as defined above. Additionally or alternatively, the reaction zone may comprise a capture molecule or capture molecules complementary to (a) part(s) of an antisense strand of the target molecule as defined above. In such a scheme, a reaction zone is capable of binding the antisense strand of a duplex nucleic acid target molecule or essentially the antisense strand of a duplex nucleic acid target molecule, or about 60%, 70%, 80%, 90%, 91%, 92%, 93%, 94%, 95%, 96%, 97%, 98%, or 99%, or 99.5% of the antisense strand of a duplex nucleic acid target molecule.

In a further embodiment of the present invention, a device as described herein above may comprise a capture zone comprising at least (a) capture molecule(s) complementary to the capture molecule(s) or a part thereof in said reaction zone. Accordingly, if a capture molecule is present in the capture zone as defined herein, a complementary capture molecule is present in the reaction zone as defined herein. Similarly, if more than one capture molecule is present in the capture zone as defined herein, the same number of complementary capture molecule is present in the reaction zone as defined herein. In further embodiments, the capture molecules in the capture zone and their complementary counterparts in the reaction zone may have identical lengths, or be substantially of the same length, or may show a degree of 70%, 80%, 85%, 90%, 95%, 96%, 97%, 98%, 99%, 99.5% or more identity in length. Capture molecules may be longer either in the capture zone, or in the reaction zone, preferably in the capture zone. Non-overlapping regions of capture molecules, i.e. the portions of the capture molecules which are not represented in the respective other zone, may preferably be located at one end or at both ends of the molecule. They may, in specific embodiments, also be located in the center of the molecule or dispersed throughout the molecule. These regions may be present as one or more than one unit, preferably as one unit at the end of the molecule.

In yet another embodiment, the number and/or length of corresponding capture molecules, i.e. of sense and antisense strand etc., may additionally or alternatively be the same, or be essentially the same in the reaction zone(s) and in the capture zone(s). For example, a capture zone may comprise any suitable coverage, such as a coverage of 1, 2, 3, 4, 5, 6, 7, 8, 9 or 10 or more copies of a capture molecule. These copies may have different lengths or be of the same length. A reaction zone may accordingly comprise any suitable coverage, such as a coverage of 1, 2, 3, 4, 5, 6, 7, 8, 9, or 10 or more copies of the corresponding complementary strand of said capture molecule. These copies may have different lengths or be of the same length. Alternatively, the number of corresponding capture molecules, i.e. of sense and antisense strand etc., may be different in the reaction zone(s) and in the capture zone(s). Thus, the coverage of capture molecules in the reaction zone may be about less than 10%, 10%, 20%, 30%, 40%, 50%, 60%, 70%, 80%, or 90% of the coverage of the complementary capture molecules in one, more or all capture zones. In further embodiments, the overage of capture molecules in the one, more or all capture zone(s) may be about less than 10%, 10%, 20%, 30%, 40%, 50%, 60%, 70%, 80%, or 90% of the coverage of the complementary capture molecules in the reaction zone(s). The coverage may, in specific embodiments, depend on the type of immobilization, the material on which the molecules are immobilized, the type of application, the envisaged use etc. Preferred is a high, excess or at least higher coverage in the capture zone(s). In particularly preferred embodiments of the present invention a pool of capture molecules may be located at a specific area of a capture zone, and subsequently be immobilized there as a mixture to the porous capture zone membrane material. The total number of capture molecules in these regions may depend on the application and be adjusted accordingly. It is furthermore preferred to have a local excess of capture molecules in one or more capture zones as defined herein.

In further embodiments, the lengths or coverage parameters between more than one reaction zone and more than one capture zone may be different or vary, i.e. there may be an increase within the device with regard to the coverage, or the coverage may be drastically increased or reduced according to suitable parameters and/or in dependence of the presence of other features, e.g. temperature conditions.

In a further embodiment of the present invention, a device as described herein above may comprise a capture zone comprising a subset of capture molecules complementary to the capture molecule(s) or a part thereof in the reaction zone. Accordingly, if a capture molecule is present in the capture zone as defined herein, a complementary capture molecule is present in the reaction zone as defined herein. However, if a capture molecule is present in the reaction zone as defined herein, a complementary capture molecule may not be present in the capture zone as defined herein. In yet another specific embodiment of the present invention a device as described herein above may comprise a reaction zone comprising a subset of capture molecules complementary to the capture molecule(s) or a part thereof in the capture zone. Accordingly, if a capture molecule is present in the reaction zone as defined herein, a complementary capture molecule is present in the capture zone as defined herein. However, if a capture molecule is present in the capture zone as defined herein, a complementary capture molecule may not be present in the reaction zone as defined herein.

The term "subset of capture molecules" as used herein refers to a number of 10%, 20%, 30%, 40%, 50%, 60%, 70%, 80%, 90%, 95%, 96%, 97%, 98%, 99%, 99.5%, 99.6%, 99.7%, or 99.8% of corresponding sense and antisense capture molecules as defined herein. The capture molecules present in both zones, as well as the capture molecules present in only one zone may be present with any suitable coverage or in any suitably copy number, e.g. 1, 2, 3, 4, 5, 6, 7, 8, 9 or 10 or more copies. Furthermore, the capture molecules may comprise variations or lengths or sequence differences as mentioned herein above.

In another embodiment, the capture zone comprises (a) capture molecule(s) capable of binding to (an) interfering sequence(s). The term "interfering sequence" as used herein refers to short repeats in the genome, e.g. transposon inverted repeats, Alu or Alu-like sequences, transposon remnants etc.

In a particularly preferred embodiment the device according to the present invention comprises at least two reaction zones, wherein one reaction zone is capable of specifically binding a sense strand(s) or part of (a) sense strand (s) of (a) target molecule(s), and the other reaction zone is capable of specifically binding (a) complementary antisense strand(s) or part of (an) antisense strand (s) of (a) target molecule(s). In this scheme the at least two reaction zones may be provided within one flow path, or may be provided in separate portions of the device. A device comprising two reactions zones as define herein may be provided with one, two or more capture zones, preferably with at least two capture zones. In embodiments, in which one capture zone is envisaged, the capture zone may be removable or replaceable with a further or different capture zone allowing the capturing of complementary or different sequences in comparison to the first capture zone. In embodiments, in which at least two capture zones are present, these capture zones may be capable of binding complementary target molecule strands. Furthermore, the capture zones may be equipped with temperature control and/or regulating units allowing for a differential heating or melting of target nucleic acid strands.

In a further preferred embodiment of the present invention one or more reaction zone(s) and one or more capture zone(s) as defined herein above, may be arranged within a device in a closed loop. The term "closed loop" as used herein refers to an arrangement of the mentioned zones, which allows a unidirectional flow of material, e.g. fluids, from one zone to the next and a corresponding returning of the material to the starting zone. Such an arrangement allows a continuous, recycling movement of material over identical zones, e.g. the one or more reaction zones, for instance via a repetition or passing of material through specific zones 2, 3, 4, 5, 6, 7, 8, 9, 10 or more times. The repetition allows in particular to increase the number of interactions between target molecules and capture molecules by repeating the opportunity of proximity between these molecules. The connection may be any suitable connection as, e.g. via tubes, pipes, pipelines etc. In such a setup, the flow rate in different parts of the device may be adjusted to the necessities. For example, a flow rate may be used which is reduced in the capture zone(s) and/or in the reaction zone(s), or only in the capture zone(s), or only in the reaction zone(s), allowing, for example, a prolonged or individually adjusted interaction window for the target molecules. The closed loop may further have one or more entry and exit points or ports, e.g. 1, 2, 3, 4 or 5 inlets or outlets. In these ports, medium or sample liquid comprising target molecules to be bound to capture molecules may be introduced or removed. In a specific embodiment, said medium or sample comprising target molecules may comprise the target molecules in a denatured or melted form as defined herein above. In further embodiments, these ports or inlets may be connected to one or more preparation or denaturing zones or chambers, wherein sample fluid or target molecules may be heated to a temperature of about 85°C to 98°C, preferably at 95°C in order to provide single stranded nucleic acid molecules. In specific embodiments, closed loops may also comprise shortcut structures allowing the disconnection of certain zones, e.g. one or more reaction or capture zones. Furthermore, the device may comprise additional outlets allowing the addition of further zones, e.g. more capture and/or reaction zones. In further embodiments, specific arrangements of loop structures may be present.

In another specific embodiment of the present invention, one or more reaction zone(s) and one or more capture zone(s) as defined herein above may be arranged within a device in a single flow path. The term "single flow path" as used herein refers to a linear arrangement of the zones without the presence of a looping element. The connection may be a connection as defined herein above, e.g. via tubes, pipes, pipelines etc. In such an arrangement, a transport of material or medium back and forth over the reaction zone and the capture zone and/or over one or more temperature control and/or regulating units as defined herein above is envisaged. Alternatively, the zones may be arranged in any other combination, as long as their linear arrangement is kept. If a single flow path is used, the flow velocity of the medium, e.g. of the hybridization solution, may be adjusted, e.g. reduced in order to allow a suitable binding interaction between capture and target molecules or strands.

In a specific embodiment of the present invention, a device as defined herein above may comprise one or more transportation means. The term "transportation means" as used herein refers to any suitable element, apparatus or unit, which allows the movement and/or transport of medium or sample or sample parts, e.g. hybridization solution, from one zone of the device to another zone of the device or vice versa, i.e. from the at least one capture zone to the at least one reaction zone. An example of such a transportation means is a pump, e.g. a 3-valve pump, a peristaltic pump or a cilia pump. However, any other types or forms of pumps, which can be suitably integrated into a device according to the present invention are also envisaged. Such a transportation means may be located between a capture zone and a reaction zone and/or may be located at the extremities of a device. Furthermore, a transportation means may be integrated in one or more zones of the device. The transportation means may preferably regulate the flow of fluids in the device. The regulation may be achieved by setting and/or adjusting the flow rate or velocity of the flow to a certain value. Such a value may be set with regard to, and made dependent on, the material to be transported, the temperature used, the target molecules or capture molecules, the type of interaction, which takes place between the target molecule and the capture molecule etc. The transportation means may further be used with different intervals of functioning. E.g., the transportation means may be used for certain period of time, e.g. for about 10 sec, 20 sec, 30 sec, 1 min, 2 min, 3 min, 5 min, 7 min, 10 min, 15 min, 30 min, 40 min, 60 min etc., subsequently be switched off for a certain period of time, e.g. for about 10 sec, 20 sec, 30 sec, 1 min, 2 min, 3 min, 5 min, 7 min, 10 min, 15 min, 30 min, 40 min, 60 min etc, subsequently be switched on again for e.g. for about 10 sec, 20 sec, 30 sec, 1 min, 2 min, 3 min, 5 min, 7 min, 10 min, 15 min, 30 min, 40 min, 60 min etc. The intervals may be changed or modified according to the preceding intervals or the stage of interaction reactions, i.e. it may become longer or shorter in subsequent steps. If more than one transportation means is present in a device, either all transportation means may work or only a sub-portion thereof. The number and location of the transportation means, which is active, may be determined and set according to the presence and localization of the reaction zone and/or the second zone.

In specific embodiments a reaction zone may be combined at one side with a transportation means, e.g. a pumping zone or chamber and may be connected on the other side with a capture zone, which in turn is followed by a transportation means, e.g. a pumping zone or chamber, or vice versa. In a further alternative, bifurcated terminal branches comprising transportation means or a zone as defined above may be used. A specific detailed version of a preferred, envisaged location and form of transportation means is depicted in Figs. 3 and 4.

In a further preferred embodiment of the present invention a device as mentioned herein above is a micro fluidic device or implemented in the form of a microfluidic device, wherein said reaction zone(s) and capture zone(s) are preferably connected in a closed loop, or are connected via a single flow path as defined above. The term "microfluidic device" as used herein refers to a device allowing the precise control and manipulation of fluids that are constrained to small, preferably sub-millimeter scales. Typically, a microfluidic device implements small volumes, e.g. in the range of nl, pl or fl, and/or it may implement a small overall size. Furthermore, a microfluidic device according to the present invention may consume a lower amount of energy. In a microfluidic device effects such as laminar flow, specific surface tensions, electrowetting, fast thermal relaxation, the presence of electrical surface charges and diffusion effects may be implemented. In certain embodiments, a microfluidic device may have connections with external sources or external elements, e.g. the separation or reservoirs or vessels for reuse purposes may be possible. Furthermore, the microfluidic device may comprise an electronic or computer interface allowing the control and manipulation of activities in the device, and/or the detection or determination of reaction outcomes or products.

In another specific embodiment of the present invention said microfluidic device may be an integrated microfluidic cartridge. The term "integrated microfluidic cartridge" as used herein refers to the compactation and resizing of a microfluidic, e.g. comprising all necessary connections, zones and, optionally, also necessary ingredients with a chamber or container like form. The cartridge may, for example, be entirely closed, or partially closed allowing the introduction of samples, ingredients etc. via resealable inlets. The cartridge may further be equipped with alignment structures for scanning or detection devices, e.g. recognition o portions for a scanner, a bar code or matrix code indicating the provided ingredients, the manufacture date etc., or an interface to a detection unit allowing electronic or optical detection of interaction between target molecules and captured molecules, e.g. in the reaction zone of a device as mentioned herein. In further preferred embodiments said cartridges may comprise on board reagent solution(s), e.g. for the purpose of single use in clinical applications, allowing to avoid cross contamination.

Within a microfluidic device according to the present invention, specific transportation means may be present. For example, a transportation means may implement a peristaltic actuation. Accordingly, flexible connections or tubing may be present in a device, or the device may be provided with one or more flexible or moveable sides or septa, allowing for a peristaltic movement. The peristaltic activity may be performed by any suitable means, e.g. a rolling device or actuator on said flexible tubing or septa, by a combination of two such tubing or septa in a contradirectional fashion, by the application of liquid mediated pressure on said tubing or septa, e.g. the provision of water filled tubing in direct contact with said tubing or septa according to the present invention, and/or by the use of a peristaltic pump. Further details and variations of this technique would be known to the person skilled in the art or could be derived from suitable textbooks. In a further embodiments, a transportation means may implement a piston and actuated membrane driving, e.g. by the employment of at least one moveable surface of a device, or of a specific region of said device. This moveable surface may accordingly be in the form of a flexible membrane and be used as a piston decreasing the volume within said device, or within said zone of said device, leading to a transportation of neighboring material outside said zone or region. The moveable surface or piston structure may be combined with suitable expansion reservoirs or zones, allowing a movement of fluids inside the device. Further details and variations of this technique would be known to the person skilled in the art or could be derived from suitable textbooks. A further example of a suitable transportation means implements a vectored vacuum, for example by the use of suitable vectored vacuum pumps sucking liquid into one or more zones of the device according to the present invention, or into a specific direction, leading to a movement of medium, e.g. hybridization solution in the device. In a specific embodiment of the present invention, it is preferred to have an average shear rate of about 1 s⁻¹ or more, which may be used to improve the binding rate. This share rate may be implemented by suitable transportation or pumping facilities, as would be known to the person skilled in the art. Further details and variations of this technique would be known to the person skilled in the art or could be derived from suitable textbooks.

In a preferred, specific embodiment of the present invention, a device as defined herein above comprises two reaction zones and two capture zones, wherein a combination of one reaction zone and one capture zone is not in fluid connection with a combination of a second reaction zone and a second capture zone and wherein each reaction zone and each capture zone comprises (a) capture molecule(s) complementary to the capture molecule(s) of the other reaction zone or capture zone. In such a scheme, a corresponding set of a sense strand capture molecule in the capture zone and an antisense strand capture molecule in the reaction zone of a part A of a device may be present, whereas a part B of a device comprises a set of an antisense strand capture molecule in the capture zone and a sense strand capture molecule in the reaction zone. This form of part A and B devices may be arranged either as single flow path device or in a closed loop. Typically, the device may be loaded with a nucleic acid target molecule containing or comprising sample, which has been proportioned into essentially equal parts, or any other suitable proportion. Furthermore, the sample may have been denatured or be present in single stranded form. Alternatively, the device may comprise an additional heating and optional separation chamber allowing for the melting or denaturation of the nucleic acid sample and optionally for its separation into essentially equal parts. Parts A and B of a device as described above may not be in fluid connection with each other.

In another preferred embodiment of the present invention a capture molecule as defined herein above, i.e. a capture molecule in the capture zone and/or in the reaction zone may have a length of about 20 to 80 nucleotides, e.g. a length of 20, 25, 30, 35, 40, 45, 50, 55, 60, 65, 70, 75, 80 or more nucleotides. In a more preferred embodiment, the capture molecule may have length of about 40 to 65 nucleotides. Examples of envisaged lengths of capture molecules include capture molecules having a length of 20, 21, 22, 23, 24, 25, 26, 27, 28,29,30,31,32,33,34,35,36,37,38,39,40,41,42,43,44,45,46,47,48,49,50,51,52, 53, 54, 55, 56, 57, 58, 59, 60, 61, 62, 63, 64, 65, 66, 67, 68, 69, 70, 71, 72, 73, 74, 75, 76, 77, 78, 79 or 80 nucleotides. Particularly preferred is a length of about 60 nucleotides, e.g. 59, 60 or 61 nucleotides. The length of a capture molecule and of its complementary counterpart in a reaction zone and in a capture zone may be identical, essentially identical or different, e.g. following the parameters and details as defined herein above. The difference in length may be made dependent on the nature, form and length etc. of the target molecule or its sequence.

In a further aspect, the present invention relates to a method of efficiently binding nucleic acids on a microarray, comprising the steps of:
(a) introducing a medium containing one or more target molecules in denatured form into a capture zone of a device as defined herein above;
(b) performing an interaction reaction between said target molecule(s) or a part thereof and immobilized capture molecule(s) in said capture zone, wherein (a) sense strand(s) or part of (a) sense strand (s) of (a) target molecule(s) is bound to said capture molecule(s), whereas (a) complementary antisense strand(s) or part of (an) antisense strand (s) of said target molecule(s) is not captured in said capture zone; or wherein (an) antisense strand(s) or part of (an) antisense strand (s) of said target molecule(s) is bound to said capture molecule(s), whereas (a) complementary sense strand(s) or part of (a) sense strand (s) of said target molecule(s) is not captured in said capture zone; and
(c) transporting not bound target molecule strand(s) to a reaction zone comprising a microarray, thereby allowing an interaction between said target molecule strand(s) or a part thereof and (an) immobilized capture molecule(s) on the microarray.

In specific embodiments of the invention, a reaction zone, a capture zone, a microarray, a capture molecule, a sense strand, an antisense strand, a target molecule, a part thereof, a medium, or the immobilization of capture molecules in the context of the methods of the present invention correspond to the definitions of these terms provided herein above in the context of a description of the device according to the present invention. Accordingly, all mentioned embodiments, variants, modifications or employments as described in the context of a device according to the present invention are to be understood as also applicable to the methods of the present invention as describe herein above or below.

The term "introducing a medium into a capture zone" as used herein refers to the provision of a medium as defined herein above in a capture zone of a device as mentioned above. This provision may be carried out by injecting said medium into the device. For example, in the capture zone itself or in an adjacent mixing zone of a device a medium may be injected from an outside or inside reservoir, or from a syringe or any other suitable receptacle. The term "introducing in a denatured form" as used herein refers to the introcution of an essentially single stranded form of the nucleic acid target molecule into the capture zone, as described herein above. Accordingly, a medium may have been brought to a melting or denaturation temperature, thus creating a high proportion of single stranded molecules. In further preferred embodiments, a medium introduced into a device according to the present invention may not be in a denatured form. In these embodiments the medium containing the target molecule(s) may, previous to the introduction into the capture zone, be heated, e.g. denatured or melted within the device as described herein above, for instance in a specific melting or heating chamber or a denaturation zone or sector as mentioned above. The heating may alternatively also be carried out in the capture zone itself, e.g. by using temperature control and/or regulating units as described herein above.

In further alternative embodiments of the present invention, a medium containing one or more target molecules may further be introduced into a different zone of a device as described herein, e.g. into a reaction zone. Accordingly, by applying suitable heat in said zone or in a different zone, the medium may be converted into a medium comprising the target molecules in a denatured form.

The term "performing an interaction reaction between said target molecule(s) or a part thereof and immobilized capture molecule(s) in said capture zone" as used herein refers to the performance of a hybridization reaction, preferably of a specific hybridization reaction. The term "specific hybridization" as used herein refers to the binding, duplexing or hybridizing of a nucleic acid to a particular further nucleic acid, e.g. a capture probe, under stringent conditions. The term "stringent condition" in the context of nucleic acid hybridization is sequence and sequence-length dependent, and may be different under different experimental parameters, as the person skilled in the art would know. Examples of stringent hybridization conditions which may be employed in the context of the present invention are a hybridization in a buffer comprising 50% formamide, 5xSSC, and 1 % SDS at 42°C, or a hybridization in a buffer comprising 5xSSC and 1% SDS at 65°C. Exemplary stringent hybridization conditions can also include a hybridization in a buffer of 40% formamide, 1 M NaCI, and 1 % SDS at 37°C. Alternatively, a hybridization may be carried out in 0.5 M NaHPO, 4.7% SDS, 1 mM EDTA at 65°C. Further additional stringent hybridization conditions include hybridization at 60°C or higher and 3xSSC (450 mM sodium chloride/45 mM sodium citrate) or incubation at 42°C in a solution containing 30% formamide, 1 M NaCI, 0.5% sodium sarcosine, 50 mM MES, pH 6.5.

In further embodiments of the present invention, said interaction may additionally comprise a washing or rising step. This step may be carried out under specific wash conditions to be used in the context of the present invention which include, for example, a salt concentration of about 0.02 molar at pH 7 and a temperature of at least about 50°C or about 55°C to about 60°C which is to be applied for about 15 minutes ; or, a salt concentration of about 0.15 M NaCl at 72°C which is to be applied for about 15 minutes; or, a salt concentration of about 0.2xSSC at a temperature of at least about 50°C or about 55°C to about 60°C which is to be applied for about 15 to about 20 minutes. In further alternatives the hybridization complex may be washed twice with a solution with a salt concentration of about 2xSSC containing 0.1% SDS at room temperature for 15 minutes and then washed twice by 0.1xSSC containing 0.1% SDS at 68°C for 15 minutes; or, equivalent conditions. Alternatively, washing may be carried out in 0.1xSSC/0.1% SDS at 68°C for about 15 min. Stringent conditions for washing can also be, for example, 0.2xSSC/0.1% SDS at 42°C.

Subsequently to the interaction of step (b) as defined herein above, not bound target molecules, preferably single stranded target molecules, more preferably single stranded target molecules without complementary strand in the medium, may be transported to a reaction zone as defined herein above. Typically, the reaction zone comprises a microarray as described herein. The "transporting" of the not bound target molecule(s) may be carried out with the help of transportation means as defined herein above. For example, a pump as defined herein above may produce a liquid flow within the device, which leads to a continuous or discontinuous flow of medium and not bound target molecules contained therein. For example, a transporting activity may comprise a continuous flow of liquids of a velocity of about 0.1 to 0.3 mm/s in a channel or tubing having a height of about 0.2 mm. Accordingly, the velocity may change or be modified in dependence of the dimension of the zone, channel, tubing or sector of a device, e.g. its height, diameter etc. Alternatively, a transporting activity may be composed of phases of liquid movement, followed by phases of non-movement, e.g. a phase of movement of 5, 10, 15, 20, 30, 40, 50 min or more may be followed by phase of non-movement of 1, 2, 3, 4, 5, 6, 7, 8, 9, 10 min or more. Furthermore, the flow velocity may be changed or modified, e.g. reduced or increased during a performance of the method.Particularly preferred is a slow flow transportation process. Further, particularly preferred is an average shear rate of about 1 to 3 s⁻¹ or more. This share rate may be implemented by suitable transportation or pumping facilities as described herein, or as would be known to the person skilled in the art.

Subsequently, in a reaction zone of the device of the present invention said not bound target molecule may be allowed to interact with an immobilized capture molecule as defined herein, i.e. with a complementary molecule or part thereof as described herein above. The term "allowing to interact" refers to the conditions of interaction, preferably hybridization conditions as used herein. The term also refers to further parameters having an influence on the interaction between the target molecule and the capture molecule within a reaction zone of the device according to the present invention. These parameters include a flow rate or flow velocity as defined herein above, the temperature at the reaction zone, the concentration of salts or further buffer ingredients as mentioned above, the amount of available capture molecules, the presence of interfering molecules in the solution, and the time of interaction etc. In further, particularly preferred embodiments of the present invention, the interaction, i.e. binding to the microarray, may be accompanied by fluid actuation, e.g. by any kind of pumping or fluid movement or transportation, for instance via a transportation means as defined herein.

In a particularly preferred embodiment, the interaction step may be carried out for a time of interaction of about 30 min to 5 h, more preferably for a time of 1 h to 3 h, e.g. for 30 min, 60 min, 1.5 h, 2 h, 2.5 h, 3 h, 3.5 h. It is emphasized that the present invention provides the advantage that the method of efficiently binding nucleic acids on a microarray may be carried out for a reduced period of time in comparison to typical hybridization methods. In particular, the presently claimed method avoids the performance of interactions for a period of time of more than 5, 10, 15 or 24 hours, e.g. overnight hybridizations or binding procedures.

In a further, preferred embodiment of the present invention the above mentioned method comprises at least one additional step (d) of detecting the interaction between said target molecule strand(s) or a part thereof and (an) immobilized capture molecule(s) on the microarray. The detection of the interaction on the microarray may be carried out according to any suitable methods or procedures known to the person skilled in the art. For example, a labeling step may be included and the presence of labeled or non-labeled molecules, the presence or absence of colors or dyes in the solution, the change of pH, and the subsequently the concentration of nucleic acids may be measured. Alternatively, electrical conductance or any other suitable parameter can be measured. This measurement may be carried out in a reaction zone of the device as described herein above. Alternatively, said detection may be carried out in a specific measurement or detection chamber or zone of a device according to the present invention, for example in a sector to which a removable reaction zone as described above may be transferred. The site of measurement may, for example, depend on the reaction type, e.g. if the reaction takes place in or on immobilized entities on the substrate leading to their detectable modification, or if it takes place in the reagent solution, leading to its detectable modification. Particularly preferred is a measurement in situ. In a particularly preferred embodiment, said detection may be carried out after a first hybridization phase, i.e. before a second capture zone is heated to release the complementary strand of a target molecule as defined herein above or below. In a particularly preferred embodiment, said detection may include a further additional detection activity after a second capture zone is heated to release said complementary strand and, in addition or optionally, the comparison of results of the first and the second detection steps, i.e. the comparison of results obtained after the first hybridization with those obtained after the second hybridization.

A corresponding detection or measurement may be carried out by optical means, e.g. via an optical read out system. For example, a CCD camera or other suitable optical equipment may be used in order to detect optical changes in a solution or at a substrate, e.g. in the reaction zone, or in the microarray and/or in order to determine the quality of such an optical change. Alternatively or additionally, a corresponding measurement may be carried out by electrical means, e.g. based on the determination of conductivity, capacity or ionic strength, pH, and/or electrophoretic parameters at a site or in a zone of the device according to the present invention. Corresponding measurement modules may be connected to an integration station, e.g. a computer, a computerized module or a microelectronic device allowing an assessment of the binding stage, the concentration of bound nucleic acid, the specificity of the binding, the binding to specific sequences or molecules etc. Such an integration station may further be equipped with suitable software or programs allowing any control activity and assessment. Such an assessment may further be used for applying corrective steps, e.g. the increase or decrease of velocity or flow parameter, the introduction of further reagent solution ingredients, the increase or decrease of the reaction temperature, the increase or decrease of a time period allocated for binding steps etc.

In further particularly preferred embodiments of methods of the present invention, said interaction reaction between said target molecule(s) and immobilized capture molecule(s) in capture zones of a device is performed in at least two capture zones wherein one capture zone is capable of specifically capturing a sense strand(s) or part of (a) sense strand (s) of (a) target molecule(s), and the other capture zone is capable of specifically capturing (a) complementary antisense strand(s) or part of (an) antisense strand (s) of (a) target molecule(s). A method may accordingly be carried out in a device subtype or variant as defined herein above, i.e. in a device comprising two capture zones.

To be operational the corresponding method should include differential hybridization/binding and denaturation/melting steps. Preferably, the method comprises a step wherein one capture zones is brought to a melting temperature or a temperature not allowing hybridizing or binding of the nucleic acid(s) to the capture molecule(s), whereas another capture zone is brought to a temperature allowing hybridizing or binding of the nucleic acid(s) to the capture molecule(s). In specific embodiment of the present invention, the method additionally or alternatively comprises a step wherein one capture zones is kept at a melting temperature or a temperature not allowing hybridizing or binding of the nucleic acid(s) to the capture molecule(s), whereas another capture zone is brought to or kept at a temperature allowing hybridizing or binding of the nucleic acid(s) to the capture molecule(s). In a further specific embodiment of the present invention, the method additionally or alternatively comprises a step wherein one capture zones is kept at or brought to a melting temperature or a temperature not allowing hybridizing or binding of the nucleic acid(s) to the capture molecule(s), whereas another capture zone is kept at a temperature allowing hybridizing or binding of the nucleic acid(s) to the capture molecule(s). In a further specific embodiment of the present invention, the method additionally or alternatively comprises a step wherein one capture zones is kept at a melting temperature or a temperature not allowing hybridizing or binding of the nucleic acid(s) to the capture molecule(s), whereas another capture zone is kept at a temperature allowing hybridizing or binding of the nucleic acid(s) to the capture molecule(s). The temperature may be set with the help of any suitable means, e.g. via the temperature control and/or regulating unit as defined in the context of the device embodiments. The time period for keeping or bringing the capture zones to the mentioned temperatures may vary in dependence of the target molecule(s), the medium used, the length of capture molecules used, the size and type of microarray etc. Further details and parameters would be known to the person skilled in the art and/or can be adapted to known methodology and suitable information derivable from textbooks or qualified literature.

It is important that the indicated temperature is arrived at or is present in the mentioned zones of the device essentially at the same time, i.e. essentially simultaneously in order to avoid a rehybridization of complementary single stranded nucleic acid molecules.

In a particularly preferred embodiment of the present invention one capture zones is kept at or brought to a melting temperature or a temperature not allowing hybridizing or binding of the nucleic acid(s) to the capture molecule(s) of about 85°C to 98°C, more preferably of about 95°C, whereas another capture zone is brought to or kept at a temperature allowing hybridizing or binding of the nucleic acid(s) to the capture molecule(s) of about 45°C to 65°C, more preferably of about 50°C.

In case more than two capture zones are used, a corresponding activity may be carried out in all capture zones.

The above indicated temperature step may be combined with or imbedded in any further step or sequence of reaction steps suitable for the binding of nucleic acid molecules. For example, the reaction zone(s) may be kept at or brought to a temperature allowing hybridizing or binding of the nucleic acids to the capture molecules of the microarray. A particularly preferred method of the present invention envisages subsequently to the mentioned steps a detection of nucleic acid target molecules bound in the reaction zone, i.e. on the microarray of a device of the invention, according to suitable detection procedures as described herein.

In another particularly preferred embodiment the methods of the invention comprise the additional step of a temperature switching between said at least two capture zones which were kept at or brought to a specific temperature as mentioned above, wherein the temperature of the capture zone with a temperature not allowing hybridizing or binding of the nucleic acids to the capture molecules is lowered to a temperature allowing hybridizing or binding of the nucleic acids to the capture molecules and wherein the temperature of the capture zone with a temperature allowing hybridizing or binding of the nucleic acid(s) to the capture molecules is raised to a temperature not allowing hybridizing or binding of the nucleic acids to the capture molecules. In a specific embodiment the methods of the invention comprise the additional step of a temperature switching between said at least two capture zones which were kept at or brought to a specific temperature as mentioned above, wherein the temperature of the capture zone with a temperature not allowing hybridizing or binding of the nucleic acids to the capture molecules is lowered to a temperature allowing hybridizing or binding of the nucleic acids to the capture molecules of about 45°C to 65°C, preferably of 50°C, and wherein the temperature of the capture zone with a temperature allowing hybridizing or binding of the nucleic acid(s) to the capture molecules is raised to a temperature not allowing hybridizing or binding of the nucleic acids to the capture molecules of about 85°C to 98°C, preferably of 95°C. This temperature switching preferably leads to the release of hitherto bound single stranded target molecules from one capture zone and the binding of complementary single stranded molecules to the other capture zone. This activity may preferably be associated with a transporting step as defined herein above, or a continuous flow activity within a device of the present invention, allowing an interaction of said hitherto bound single stranded target molecules to (a) capture molecule(s) in the microarray of the present invention. Variations and modifications of this switching activity are also envisaged by the present invention, e.g. in dependence of the microarray present in the device, the nature and number of reaction zone(s) etc. A particularly preferred method of the present invention envisages subsequently to the mentioned steps a detection of nucleic acid target molecules bound in the reaction zone, i.e. on the microarray of a device of the invention, according to suitable detection procedures as described herein.

In a specific embodiment of the present invention, the following sequence of activity steps may be carried out: (1) a first capture zone as defined herein above is brought to a melting temperature as described above, (2) a single strand target molecule is interacted with another, second capture zone as defined herein above, (3) a complementary single strand target molecule is interacted with a reaction zone as defined herein above, (4) said first capture zone is cooled down, (5) a second capture zone as defined herein above is brought to a melting temperature as described above, (6) thereby the previously interacted single stranded target molecule is released, (7) the complementary single stranded target molecule of step (3) is interacted with said first capture zone and (8) said single target molecule of step (2) is interacted with said reaction zone as defined herein above.

The depicted sequence may be varied or modified as mentioned herein above. For example, there may be a second reaction zone, the capture zones may be separated, steps may be repeated, other steps may be avoided etc.

In yet another preferred embodiment of the present invention a method as defined herein above may additionally comprising as step (e) a subsequent or secondary activity once a nucleic acid has been bound efficiently to a reaction zone, e.g. to the microarray according to the present invention. Such an activity comprises, for example, the amplification of (a) target molecule(s) bound to (a) capture molecule(s) in the reaction zone. Such an amplification may be carried out according to any suitable means known to the person skilled in the art. For example, the amplification may be performed on the basis of polymerase chain reaction (PCR) procedures, as derivable from qualified textbooks or literature. Alternatively, isothermal amplification processes such as NASBA or RCA may be used.

A further preferred activity is the labeling of target molecule(s) bound to (a) capture molecule(s) in said reaction zone. Such a labeling activity may be based on conjugation reactions using various chemistries, e.g. aldehyde, carboxylic acids, or amine reactions. Examples of labels that can be conjugated to a nucleic acid target molecule include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). Further labels which may be used within the context of the present invention include 6-FAM, HEX, TET, ROX, Cy3, Cy5, Texas Red or Rhodamine, TAMRA, Dabcyl, Black Hole Quencher, BHQ-1 or BHQ-2. Target molecules may also be labeled with radioisotopes e.g. ³H, ¹⁴C, ³²P, ³³P, ³⁵S, ¹²⁵I, ¹¹C, ¹³N, ¹⁵O, ¹⁸F, ⁶⁴Cu, ⁶²Cu, ¹²⁴I, ⁷⁶Br, ⁸²Rb, ⁶⁸Ga or ¹⁸F.

A further preferred activity is the ligation of target molecule(s) bound to (a) capture molecule(s) in said reaction zone. The ligation process may be carried out between target molecule(s) bound to (a) capture molecule and any suitable further nucleic acid molecule, e.g. an adaptor molecule, a tag comprising nucleic acid, a label comprising nucleic acid, a PCR-binding site comprising nucleic acid, a bar-coded sequences identification purposes, a restriction nuclease recognition site comprising nucleic acid, a nucleic acid comprising or encoding a enzymatic or protein functionality etc. For the ligation process a preparation step for the bound target molecule may be carried out, e.g. the addition of the complementary strand of a molecule in order to obtain a duplex nucleic acid molecule and/or a restriction by a restriction nuclease. The ligation may be performed with the help of enzymatic activities such as DNA ligase, e.g. DNA ligase derived from prokaryotic or eukaryotic sources.

A further preferred activity is the performance of single base extension with (a) target molecule(s) bound to (a) capture molecule(s) in said reaction zone. A single base extension may be used for the determination of the identity of a nucleotide base at a specific position along a nucleic acid or target nucleic acid molecule. The method is typically used to identify a single nucleotide polymorphisms. The single based extension may be carried out by hybridizing an oligonucleotide primer to a complementary region along a nucleic acid bound to a capture molecule, thus forming a duplex structure, with the primer's terminal 3' end directly adjacent to the nucleotide base to be identified. The oligonucleotide primer may subsequently be extended enzymatically by a single base nucleotide terminator complementary to the nucleotide being identified. In further embodiments, the identity of the terminator may be determined, e.g. via fluorescence labeling, mass labeling for mass spectrometry, measuring enzyme activity using a protein moiety, or isotope labeling. Further details and suitable procedures would be known to the person skilled in the art or can be derived from suitable textbooks or qualified literature. The corresponding method or procedure may accordingly be used for the determination of single nucleotide polymorphisms in bound target molecule.

Another preferred activity is the performance of a sequencing reaction with (a) target molecule(s) bound to (a) capture molecule(s) in said reaction zone. Such a sequencing reaction may be based on one or more reactions used in methods for sequence determination, as known to the person skilled in the art. The methods may include a release of bound target molecule(s), the preparation of duplex nucleic acids molecules, corresponding specific preparation steps or may, alternatively, be carried out in situ. Sequencing reactions as envisaged by the methods of the present invention include inter alia next generation sequencing methods or high throughput sequencing methods, for example a pyrosequencing based on the technique provided by Roche for the 454 Genome Sequencer. This method amplifies DNA inside water droplets in an oil solution with each droplet containing a single DNA template attached to a single primer-coated bead that then forms a clonal colony. Pyrosequencing uses luciferase to generate light for detection of the individual nucleotides added to the nascent DNA, and the combined data are used to generate sequence read-outs. Another example is the methodology of Illumina or Solexa sequencing, which is based on reversible dye-terminators. DNA molecules are typically attached to primers on a slide and amplified so that local clonal colonies are formed. Subsequently one type of nucleotide at a time may be added, and non-incorporated nucleotides are washed away. Subsequently, images of the fluorescently labeled nucleotides may be taken and the dye is chemically removed from the DNA, allowing a next cycle. A further example is the Applied Biosystems' SOLiD technology, which employs sequencing by ligation. This method is based on the use of a pool of all possible oligonucleotides of a fixed length, which are labeled according to the sequenced position. Such oligonucleotides are annealed and ligated. Subsequently, the preferential ligation by DNA ligase for matching sequences typically results in a signal informative of the nucleotide at that position. Since the DNA is typically amplified by emulsion PCR, the resulting bead, each containing only copies of the same DNA molecule, can be deposited on a glass slide resulting in sequences of quantities and lengths comparable to Illumina sequencing. Yet another example is Helicos' Heliscope technology, wherein fragments are captured by polyT oligomers tethered to an array. At each sequencing cycle, polymerase and single fluorescently labeled nucleotides are added and the array is imaged. The fluorescent tag is subsequently removed and the cylce is repeated. Further examples of sequencing techniques encompassed within the present invention are microfluidic Sanger sequencing. The present invention also envisages further developments of these techniques, e.g. further improvements of the accuracy of the sequence determination, or the time needed for the determination of the genomic sequence of an organism etc. These sequencing methods may be adapted within the presently provided methodology, allowing for a highly increased reading length and reduced costs due to the possibility of increasing the amount of enzymes and/or oligomers and/or nucleotides during the performance of the reactions. Further variants and details of the methods would be known to the person skilled in the art, and can be derived from suitable publications e.g. Metzger, 2010, Nature Reviews, Genetics, Vol 11.

In yet another aspect the present invention relates to the use of a device as defined herein above or of a method as defined herein above for efficiently binding a target molecule(s) on a microarray. The term "efficient binding" as used in this context refers to the performance of interaction reactions between a capture molecule in the reaction zone and a target molecule as defined herein.

Alternatively, a device or a method as defined herein above may be used for enriching one or more target molecule. The term "enriching" as used herein refers to the increase of concentration of a target nucleic acid. Further envisaged is the capturing of more than one copy of a target nucleic acid.

Furthermore, a device or a method as defined herein above may be used for specifically selecting one or more target molecules. The term "specifically selecting" as used herein refers to an interaction between elements or entities of the device according to the invention and target molecules as defined herein, in particular an interaction based on hydrogen-bonding and/or an interaction based on electric charges, e.g. between differently charged molecules. By specifically selecting a target molecule, single or multiple mismatches or differences between a capture molecule and a target molecule may be detected. This approach may be used for the detection of single nucleotide polymorphisms, or for the detection of mutated or modified nucleic acid target molecules.

Moreover, a device or a method as defined herein above may be used for quantitative and/or multiplexed detection of (a) DNA or RNA molecule(s). Corresponding procedures would be known to the person skilled in the art, including the performance of suitable amplification and/or labeling steps. In case RNA molecules are involved, the presence of RNAse inhibitors is envisaged. Furthermore, an amplification step leading to the production of DNA molecules may be added.

Also envisaged is the use of a device or a method as defined herein above for expression analysis, comparative genomic hybridization, the detection of single nucleotide polymorphisms, or for microarray-based genomic selection-based sequencing. Corresponding molecular techniques are known to the person skilled in the art. These techniques may be derived from suitable textbooks or qualified literatures. The procedures may further be adapted to the situation of the captured molecules, i.e. a specific release or preparation step may be included, or the methodology may be adapted to in situ performance on a microarray as define herein.

Further envisaged is the use of the device as defined herein above for any other type of suitable molecular reactions as mentioned herein or known to the person skilled in the art.

The following example and figures are provided for illustrative purposes. It is thus understood that the example and figures are not to be construed as limiting. The skilled person in the art will clearly be able to envisage further modifications of the principles laid out herein.

### EXAMPLES

### Example 1 - Effect of storage time between denaturing and injection on microarray

A sample obtained by PCR amplification of a short sequence of a human genome was diluted to 1 nM in hybridization buffer. It was heated in a bath to 95°C for 5 min and then put on ice and stored at 50°C. After a period indicated as storage time the solution was injected into a device containing a micro-array with capture probes complementary to one of the two complementary strands of the PCR product. The probe length was 76 bp. The capture probes were immobilized to the surface by UV cross-linking. The substrate was glass with an amino-silane surface layer. The sample was introduced into the microfluidic channel covering the micro-array with a syringe pump at a rate of 6 µl/min. The cannel dimensions were 1200 x 100 µm. The flow was continued for 30 min at this rate. The micro-array was set at a temperature of 50°C for hybridization. After cessation of flow a washing buffer was pumped through at a rate of 6 µl/min for 5 min. Then the microarray was placed on a confocal fluorescence scanner and the intensity of the spots was measured.

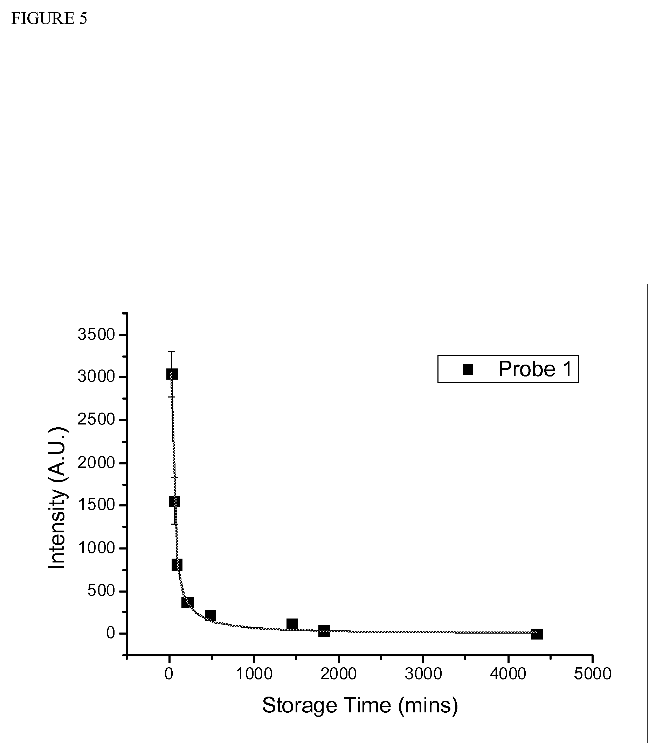

Figure 5 shows the measured intensity of the spots for the different experiments using different storage times. As can be seen the intensity drops by a factor of 10 due to storage. This indicates that the effective concentration of single stranded molecules which are available for hybridization to the microarray decreases by at least a factor of 10 (the concentration dependence of the signal is sublinear).

Since during every hybridization experiment rehybridization in solution occurs even with direct injection after denaturing the capture efficiency is compromised. For capture of low concentration targets long hybridization times are common practice, typically over night and up to 3 days (corresponding to the most right point in Figure 5). Consequently, removing the complementary strands in solution as described herein above can improve the capture efficiency of a microarray up to a factor of 10.

## Claims

1. A device for the efficient binding of nucleic acids on a microarray, comprising:
(a) at least one reaction zone comprising a microarray, wherein the microarray comprises a substrate, on which one or more species of capture molecules are immobilized, and
(b) at least one capture zone comprising a porous membrane substrate, on which one or more species of capture molecules are immobilized, which is in fluid connection with the reaction zone;
wherein said capture zone is capable of specifically capturing (a) sense strand(s) or part of (a) sense strand (s) of (a) target molecule(s), whereas (a) complementary antisense strand(s) or part of (an) antisense strand (s) of said target molecule(s) can be captured in said reaction zone; or wherein said capture zone is capable of specifically capturing (an) antisense strand(s) or part of (an) antisense strand (s) of said target molecule(s), whereas (a) complementary sense strand(s) or part of (a) sense strand (s) of said target molecule(s) can be captured in said reaction zone.

2. The device of claim 1, wherein said device comprises at least two capture zones wherein one capture zone is capable of specifically capturing (a) sense strand(s) or part of (a) sense strand (s) of (a) target molecule(s), and the other capture zone is capable of specifically capturing (a) complementary antisense strand(s) or part of (an) antisense strand (s) of (a) target molecule(s).

3. The device of claim 1 or 2, wherein said device comprises at least one temperature control and/or regulating unit for controlling and/or regulating the temperature within the device, preferably located in said capture zone, and optionally also located in said reaction zone.

4. The device of claim 3, wherein one of the at least two capture zones is kept at or brought to a melting temperature or a temperature not allowing hybridizing or binding of the nucleic acid(s) to the capture molecules, preferably a temperature of about 85°C to 98°C, whereas another capture zone is kept at or brought to a temperature allowing hybridizing or binding of the nucleic acid(s) to the capture molecules, preferably a temperature of about 45°C to 65°C.

5. The device of any one of claims 1 to 4, wherein said reaction zone comprises (a) capture molecule(s) complementary to the sense and anti-sense strand(s) or parts thereof of the target molecule(s), or (a) capture molecule(s) complementary to only the sense strand(s) or part of (a) sense strand (s) of the target molecule(s), or (a) capture molecule(s) complementary to only the antisense strand(s) or part of (an) antisense strand (s) of the target molecule(s).

6. The device of any one of claims 1 to 5, wherein said capture zone comprises at least (a) capture molecule(s) complementary to the capture molecule(s) or a part thereof in said reaction zone; or comprises a subset of capture molecules complementary to the capture molecule(s) or a part thereof in said reaction zone; and/or comprises (a) capture molecule(s) capable of binding to (an) interfering sequence(s), and/or wherein the device comprises at least two reaction zones wherein one reaction zone is capable of specifically binding a sense strand(s) or part of (a) sense strand (s) of (a) target molecule(s), and the other reaction zone is capable of specifically binding (a) complementary antisense strand(s) or part of (an) antisense strand (s) of (a) target molecule(s).

7. The device of any one of claims 1 to 6, wherein said reaction zone(s) and said capture zone(s) are arranged in a closed loop, preferably in an integrated device with said reaction zone(s) and said capture zone(s) in a connected microfluidic system, or wherein said device comprises two reaction zones and two capture zones, wherein a combination of one reaction zone and one capture zone is not in fluid connection with a combination of a second reaction zone and a second capture zone and wherein each reaction zone and each capture zone comprises (a) capture molecule(s) complementary to the capture molecule(s) of the other reaction zone or capture zone.

8. The device of any one of claims 1 to 7, wherein said capture molecule has a length of about 20 to 80 nucleotides, preferably of about 40 to 65, more preferably of about 60 nucleotides.

9. The device of any one of claims 1 to 8, wherein said porous membrane substrate is a fabric or felt, and/or comprises a random porous structure such as nylon, or comprises a regular porous structure, preferably with 3-dimensional and/or 2-dimensional pore structures, such as anisotropically etched Al-oxide, or electro-spun fibers, or wherein said porous membrane substrate is created in situ, preferably by polymerization induced phase separation.

10. A method of efficiently binding nucleic acids on a microarray, comprising the steps of:
(a) introducing a medium containing one or more target molecules in denatured form into a capture zone of a device as defined in any one of claims 1 to 9;
(b) performing an interaction reaction between said target molecule(s) or a part thereof and immobilized capture molecule(s) in said capture zone, wherein (a) sense strand(s) or part of (a) sense strand (s) of (a) target molecule(s) is bound to said capture molecule(s), whereas (a) complementary antisense strand(s) or part of (an) antisense strand (s) of said target molecule(s) is not captured in said capture zone; or wherein (an) antisense strand(s) or part of (an) antisense strand (s) of said target molecule(s) is bound to said capture molecule(s), whereas (a) complementary sense strand(s) or part of (a) sense strand (s) of said target molecule(s) is not captured in said capture zone; and
(c) transporting not bound target molecule strand(s) to a reaction zone comprising a microarray, thereby allowing an interaction between said target molecule strand(s) or a part thereof and (an) immobilized capture molecule(s) on the microarray.

11. The method of claim 10, additionally comprising as step (d) detecting the interaction between said target molecule strand(s) or a part thereof and (an) immobilized capture molecule(s) on the microarray, preferably via an optical read out system.

12. The method of claim 10 or 11, wherein said interaction reaction between said target molecule(s) and immobilized capture molecule(s) is performed in at least two capture zones wherein one capture zone is capable of specifically capturing a sense strand(s) or part of (a) sense strand (s) of (a) target molecule(s), and the other capture zone is capable of specifically capturing (a) complementary antisense strand(s) or part of (an) antisense strand (s) of (a) target molecule(s) and wherein one capture zones is kept at or brought to a melting temperature or a temperature not allowing hybridizing or binding of the nucleic acid(s) to the capture molecule(s), preferably a temperature of about 85°C to 98°C, whereas another capture zone is kept at or brought to a temperature allowing hybridizing or binding of the nucleic acid(s) to the capture molecule(s), preferably a temperature of about 45°C to 65°C.

13. The method of claim 12, additionally comprising the step of a temperature switching between said at least two capture zones, wherein the temperature of the capture zone with a temperature not allowing hybridizing or binding of the nucleic acids to the capture molecules is lowered to a temperature allowing hybridizing or binding of the nucleic acids to the capture molecules, preferably a temperature of about 45°C to 65°C, and wherein the temperature of the capture zone with a temperature allowing hybridizing or binding of the nucleic acid(s) to the capture molecules is raised to a temperature not allowing hybridizing or binding of the nucleic acids to the capture molecules, preferably a temperature of about 85°C to 98°C.

14. The method of any one of claims 10 to 13, additionally comprising as step (e) an activity selected from the group comprising:
(1) amplifying of (a) target molecule(s) bound to (a) capture molecule(s) in said reaction zone;
(2) labeling of (a) target molecule(s) bound to (a) capture molecule(s) in said reaction zone;
(3) ligating of (a) target molecule(s) bound to (a) capture molecule(s) in said reaction zone to another nucleic acid;
(4) performing single base extension with (a) target molecule(s) bound to (a) capture molecule(s) in said reaction zone; and
(5) sequencing of (a) target molecule(s) bound to (a) capture molecule(s) in said reaction zone to another nucleic acid.

15. Use of a device as defined in any one of claims 1 to 9 for efficiently binding a target molecule(s) on a microarray, for enriching (a) target molecule(s), or for specifically selecting (a) target molecule(s), preferably for quantitative and/or multiplexed detection of (a) DNA or RNA molecule(s), or for expression analysis, comparative genomic hybridization, the detection of single nucleotide polymorphisms, or for microarray-based genomic selection-based sequencing.
